(19) Europäisches Patentamt
European Patent Office
Office européen des brevets

(11) Publication number: 0 278 176 B1

(12) **EUROPEAN PATENT SPECIFICATION**

(45) Date of publication of patent specification: **09.03.94**

(51) Int. Cl.5: **C07D 311/86**, C07D 311/78, A61K 31/35

(21) Application number: **87311274.2**

(22) Date of filing: **22.12.87**

(54) **Compounds having antitumour and antibacterial properties.**

(30) Priority: **23.12.86 NZ 218781**

(43) Date of publication of application:
**17.08.88 Bulletin 88/33**

(45) Publication of the grant of the patent:
**09.03.94 Bulletin 94/10**

(84) Designated Contracting States:
**AT BE CH DE ES FR GB GR IT LI LU NL SE**

(56) References cited:

**CHEMICAL ABSTRACTS, vol. 76, 1972, page 478, abstract no. 126784w, Columbus, Ohio, US; & JP-A-72 00 425 (YOSHITOMI PHARMACEUTICAL INDUSTRIES, LTD) 07-01-1972**

(73) Proprietor: **WARNER-LAMBERT COMPANY**
**201 Tabor Road**
**Morris Plains New Jersey 07950(US)**

(72) Inventor: **Denny, William Alexander**
**165 Gossamer Drive**
**Pakuranga**
**Auckland(NZ)**
Inventor: **Baguley, Bruce Charles**
**36a Olsen Avenue**
**Hillsborough**
**Auckland 4(NZ)**
Inventor: **Atwell, Graham John**
**37 Hawkins Street**
**Meadowbank**
**Auckland 5(NZ)**
Inventor: **Rewcastle, Gordon William**
**24 Rothery Road**
**Manurewa**
**Auckland(NZ)**

(74) Representative: **Senior, Janet et al**
**Abel & Imray**
**Northumberland House**
**303-306 High Holborn**
**London WC1V 7LH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 278 176 B1

## Description

The present invention relates to novel xanthenone-4-acetic acids having antitumour and antibacterial properties, to methods of preparing the novel compounds, and to the use of these compounds as antitumour and antibacterial agents.

Xanthenone-1-acetic acid has been prepared previously (H Nichino and K Kurosawa, Bull. Soc. Chem. Jap., 1983, 56, 2847), and has been evaluated as an antiinflammatory agent (M Nakanishi, T Oe, M Tsuruda, H Matsuo, S Sukuragi, Y Maruyama, Yakugaki Zasshi, 1976, 96, 99 ; Chem. Abstr. 1976, 84, 135515b). Xanthenone-2-acetic acid has also been reported previously (M Henryk, Pol. J. Chem., 1980, 54, 2059) and has been evaluated as an antiinflammatory agent (M Nakanishi, T Oe, Y Maruyama, Ger. Offen. 2,015.265; Chem. Abstr. 74P, 13008m : M Nakanishi, T Oe, S Kakuragi, Japan 73 26 767; Chem. Abstr. 78P, 159430x). Xanthenone-4-acetic acid has been prepared previously (H Nichino and K Kurosawa, Bull. Soc. Chem. Jap., 1983, 56, 2847) and has been evaluated as an anti-inflammatory agent (M Nakanishi, T Oe, M Tsuruda, H Matsuo, S Sakuragi, Y Maruyama, Yakugaku Zasshi, 1976, 96, 99; Chem. Abstr. 1976, 84 135515b; M Nakanishi, T Oe, Y Maruyama, Japan 72 00 425; Chem. Abstr. 1972, 76P, 126784w).

We have now found that the known xanthenone-4-acetic acid and xanthenone-4-acetic acids falling within the scope of formula (I) hereinafter have antibacterial and antitumour properties and are useful as antibacterial and antitumour agents.

In one aspect the present invention relates to the class of xanthenone-4-acetic acids represented by the general formula (I)

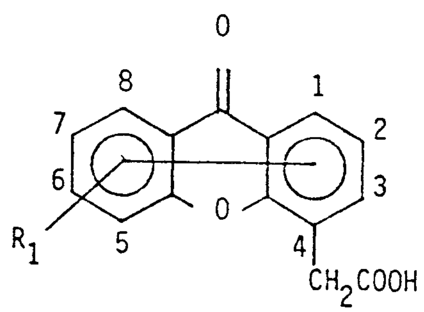

(I)

where $R_1$ represents up to two of the groups lower alkyl, halogen, phenyl $CF_3$, CN, $NO_2$, $NH_2$,$CH_2COOH$, $OR_2$, OH, $NHCOR_2$, $NHSO_2R_2$, $SR_2$, $SO_2R_2$, $CH_2CONHR_2$ or $NHR_2$ (where $R_2$ is lower alkyl optionally substituted with hydroxy, amino or methoxy functions), at any of the positions 1-8 which are available, $R_1$ may also represent the substitution of an aza (-N=) group for one or two of the methine (-CH=) groups in the carbocyclic rings and two of $R_1$ on any two available adjacent positions may also represent the grouping -CH=CH-CH=CH- to form an additional fused benzene ring;
and basic addition salts thereof.

In compounds of the formula (I) $R_1$ represents

(a) up to two substituents, which may be the same or different, selected from lower alkyl, phenyl, halogen, $CF_3$, CN, $NO_2$, $NH_2$, $CH_2COOH$, $OR_2$, OH, $NHCOR_2$, $NHSO_2R_2$, $SR_2$, $SO_2R_2$, $CH_2CONHR_2$ and $NHR_2$, and/or

(b)

(i) up to two replacements of ring -CH= by -N= or

(ii) the grouping -CH=CH-CH=CH- on adjacent ring carbon atoms, or

(iii) up to two replacements of ring -CH= by -N= and the grouping -CH=CH-CH=CH-.

Compounds in which $R_1$ represents up to two substituents in total selected from lower alkyl, phenyl, halogen, $CF_3$, CN, $NO_2$, $NH_2$, $CH_2COOH$, $OR_2$, OH, $NHCOR_2$, $NHSO_2R_2$, $SR_2$, $SO_2R_2$, $CH_2CONHR_2$, $NHR_2$ and -N= (as replacement for -CH=), or represents the grouping -CH=CH-CH=CH- on adjacent ring carbon atoms should especially be mentioned.

Compounds having one or two aza groups or having one or two other monovalent substitutions or the bivalent substituent -CH=CH-CH=CH- should also be mentioned.

$R_2$ represents a lower alkyl radical or a lower alkyl radical substituted by one or more of the same or different substituents selected from hydroxy, amino and methoxy functions.

An amino substituent of a lower alkyl radical represented by $R_2$ may be unsubstituted or, for example, substituted by one or two lower alkyl groups (where lower alkyl has the meaning given below), especially by

one or two ethyl groups. Thus, for example, an amino substituent of a lower alkyl radical represented by $R_2$ may be $NH_2$, $NHCH_3$ or $N(CH_3)_2$.

Preferably a lower alkyl radical represented by $R_2$ is unsubstituted or substituted by one or more of the same functions. Lower alkyl radicals which are unsubstituted or mono-substituted should especially be mentioned.

In the specification the term "lower" applied with reference to alkyl groups in the formula or to alkylamines denotes an alkyl group having from 1 to 5 carbon atoms. Thus, when either $R_1$ or $R_2$ represents lower alkyl, the group may contain from 1 to 5 carbon atoms.

The compounds of formula (I) form salts, including pharmaceutically acceptable salts with both organic and inorganic bases. Examples of suitable bases for salt formation include alkali metal hydroxides and carbonates, ammonia and lower alkylamines where the alkyl group may be optionally substituted with one or more hydroxy groups and may contain from 1 to 5 carbon atoms.

The xanthenone-4-acetic acids of general formula (I) and the salts thereof may be prepared, for example, by a process which comprises cyclodehydrating a substituted phenoxybenzoic acid of the general formula (IV)

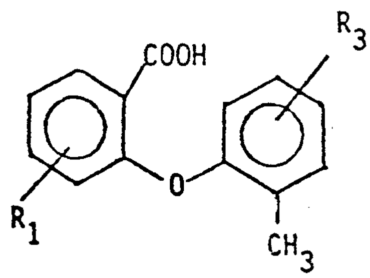

(IV)

where $R_1$ is hydrogen or is $R_1$ as defined for formula (I) and $R_3$ is as defined for $R_1$, not more than one of $R_1$ and $R_3$ being hydrogen; brominating the obtained 4-methylxanthenone of the general formula (VI)

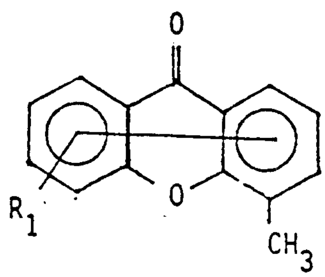

(VI)

reacting the obtained 4-bromomethylxanthenone of the general formula (VII)

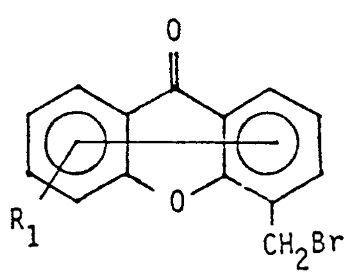

(VII)

with an inorganic cyanide, and hydrolysing the obtained xanthenone-4-acetonitrile of the general formula (VIII)

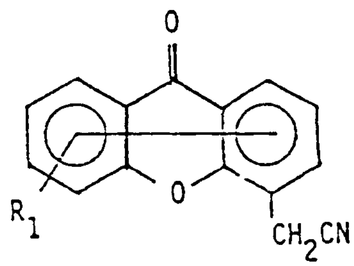

(VIII)

to the corresponding compound of formula (I) and, if desired, converting the compound of formula (I) to a basic addition salt thereof.

The compounds of general formula (I) and the salts thereof may be more directly prepared by a process which comprises cyclodehydrating a substituted phenoxyphenylacetic acid of the general formula (XI)

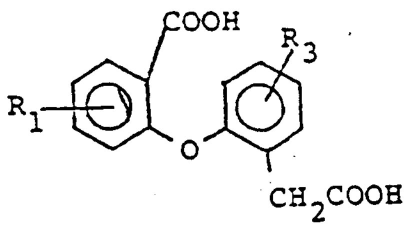

(XI)

where $R_1$ is hydrogen or is $R_1$ as defined for formula (I) and $R_3$ is as defined for $R_1$, not more than one of $R_1$ and $R_3$ being hydrogen, to the corresponding compound of formula (I) and, if desired, converting the compound of formula (I) into a basic addition salt thereof.

The xanthenone-4-acetic acids of general formula (I) and the salts thereof may further be prepared by a process which comprises cyclodehydrating a compound of the general formula (XVIII)

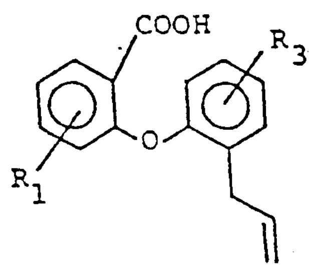

(XVIII)

where $R_1$ is hydrogen or is $R_1$ as defined for formula (I) and $R_3$ is as defined for $R_1$, not more than one of $R_1$ and $R_3$ being hydrogen; oxidising the obtained 4-allylxanthenone of the general formula (XIX)

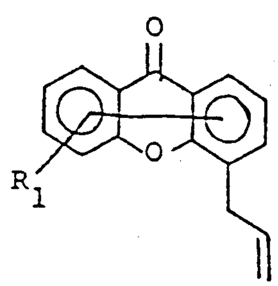

(XIX)

to the corresponding compound of formula (I) and, if desired, converting the compound of formula (I) into a basic addition salt thereof.

Compounds of the general formula (I), wherein $R_1$ is in the 5-position, and the salts thereof may be prepared by a process which comprises subjecting a compound of the general formula (XXII)

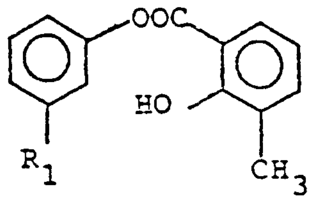

(XXII)

wherein $R_1$ is as defined for formula (I), to controlled pyrolysis, brominating the obtained 4-methylxanthenone of the general formula (XXIII)

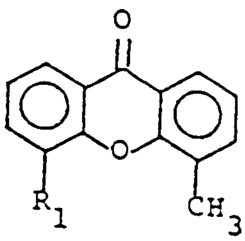

(XXIII)

reacting the obtained 4-bromomethylxanthenone with an inorganic cyanide; and hydrolysing the obtained xanthenone-4-acetonitrile to the corresponding compound of formula (I) and, if desired, converting the compound of formula (I) to a basic addition salt thereof.

The above processes for preparing the compounds of the invention, and examples of the preparation of the respective starting materials, are outlined in the following Schemes I - IV.

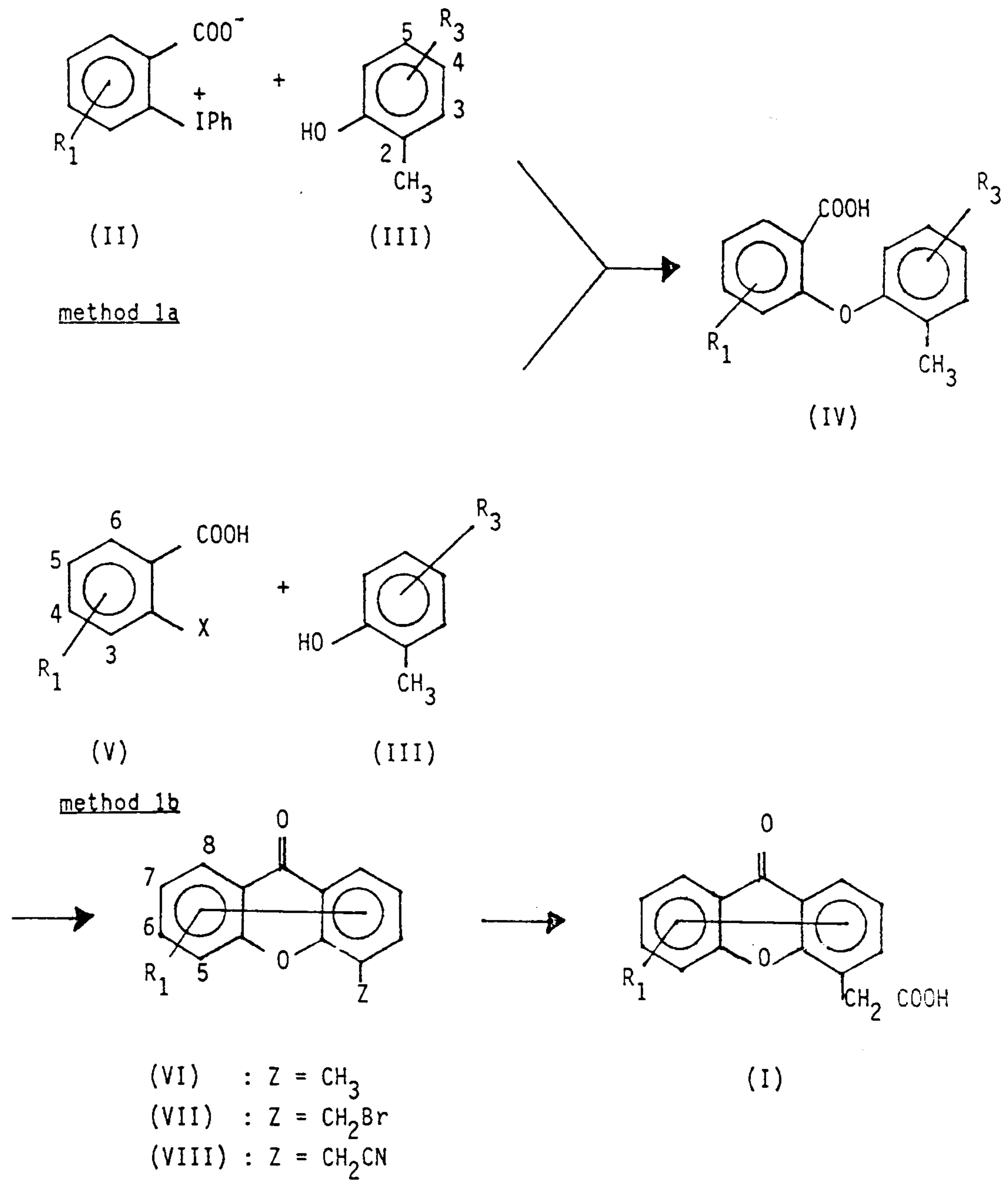

(II) (III)

method 1a

(IV)

(V) (III)

method 1b

(VI) : Z = $CH_3$

(VII) : Z = $CH_2Br$

(VIII) : Z = $CH_2CN$

(I)

SCHEME I

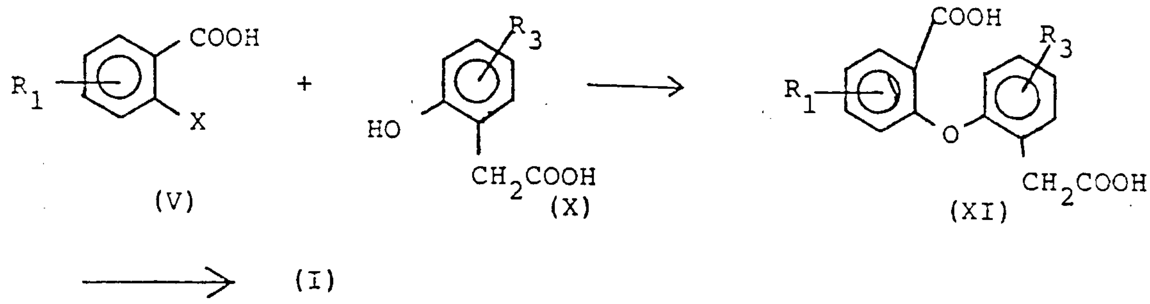

(V) (X) (XI) (I)

method 2a

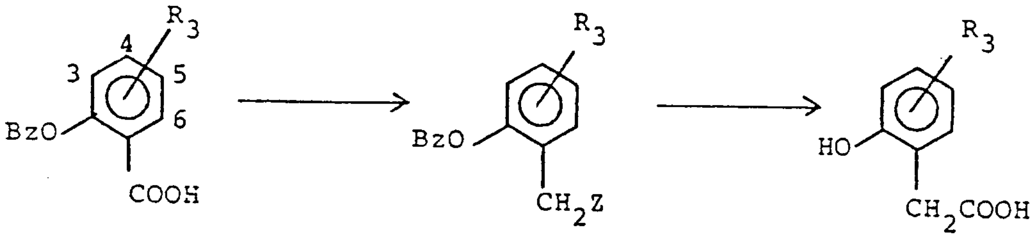

(XII)

(XIII) : Z = OH

(XIV) : Z = halogen

(XV) : Z = CN

(X)

method 2b

SCHEME II

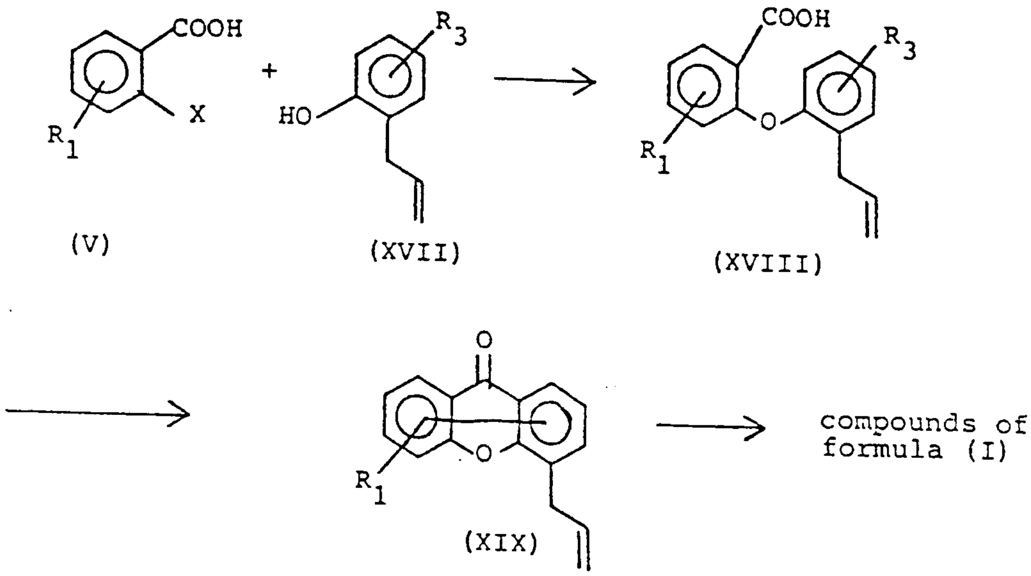


(V) (XVII) (XVIII) (XIX)

SCHEME III

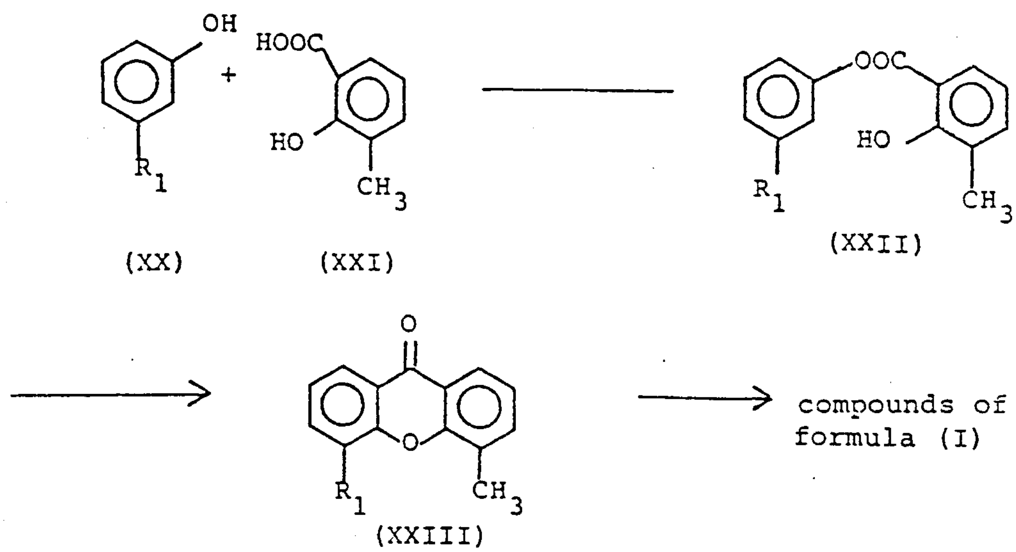


(XX) (XXI) (XXII) (XXIII)

SCHEME IV

In Scheme I, $R_1$ is hydrogen or is $R_1$ as defined for formula (I), $R_3$ is as defined for $R_1$, and X is halogen. Reaction of either diphenyliodonium-2-carboxylates (II) or halobenzoic acids (V) with phenols (III) gives the phenoxybenzoic acids (IV) in moderate yields. Reactions using diphenyliodonium-2-carboxylates (method 1a) are typically carried out at 90-140 °C for 5-10 hours in either excess phenol as solvent or DMF as cosolvent. Reactions using halobenzoic acids (method 1b) are typically carried out at 100-180 °C for 5-10

hours, using dipolar aprotic solvents (eg DMF, N-methylpyrrolidone, DMSO, HMPT, or preferably anisole or dioxan), together with 10 mole-% of CuCl and 10 mole-% of tris-[2-(2-methoxyethoxy)ethyl]amine (TDA-1, Aldrich Chemical Co).

The phenoxybenzoic acids (IV) are cyclized to the methylxanthenones (VI) using a variety of cyclodehydrating conditions (eg. 90-100% sulfuric acid, methanesulfonic acid, polyphosphoric acid, polyphosphate ester). Free-radical bromination of the methylxanthenones using bromine carriers such as N-bromosuccinimide or N-bromomaleimide and a radical initiator gives the bromomethyl compounds (VII), which undergo substitution reactions with inorganic cyanides to give the acetonitriles (VIII). Hydrolysis of these with aqueous acid then gives the desired xanthenoneacetic acids (I).

In Scheme II, $R_1$ and $R_3$ are as defined above and X is halogen. Reaction of halobenzoic acids (V) with hydroxyphenylacetic acids (X) (method 2a) using the conditions described above for method 1b gives the phenoxyphenylacetic acids (XI) in moderate to good yields, and these can be cyclized by the methods given above to give the desired xanthenone-4-acetic acid (I) directly.

Substituted 2-hydroxyphenylacetic acids (X) are conveniently prepared from available substituted salicylic acids by method 2b.

The substituted salicylic acids are treated with excess benzyl bromide under phase-transfer catalysis (tetrabutylammonium bromide), followed by treatment with alkali to hydrolyse the benzyl ester, to give the O-protected salicylic acids (XII). The corresponding acid chlorides are then made using thionyl chloride, and are reduced with sodium borohydride to the alcohols (XIII). These are converted to the halides (XIV, Z = Cl or Br) by treatment with the appropriate phosphorus trihalide, and then to the acetonitrile (XV) with NaCN under phase-transfer catalysis as above. Alkaline hydrolysis is then used to provide the O-protected acetic acid, which is not purified but deprotected by hydrogenation to give the desired substituted 2-hydroxyphenylacetic acids (X).

In Scheme III, $R_1$ and $R_3$ are as defined above, and X is halogen. Reaction of halobenzoic acids (V) with 2-allylphenols (XVII) using the conditions described above for method 1b gives the ethers (XVIII) in moderate to good yields, and these can be cyclized by the methods given above to give the 4-allylxanthenones (XIX). Controlled oxidation of the allyl group using $KMnO_4$ or other oxidants gives the desired xanthenone-4-acetic acids (I).

In Scheme IV, $R_1$ is as defined above for formula (I). Condensation of the appropriate phenol (XX) with 3-methylsalicylic acid (XXI) gives the esters (XXII) which on controlled pyrolysis yield the 4-methylxanthenones (XXIII) in low to moderate yield. These compounds can then be elaborated by the above methods to the compounds of formula (I).

As will be appreciated, the compounds shown/described in the specification may, if appropriate, be in the form of salts, and one compound of the invention prepared by a process described may be converted if desired into another such compound; for example, a compound of the formula (I) may be converted into a salt thereof, or vice-versa.

The following Tables I and II set out physical data for the known compound xanthenone-4-acetic acid and for compounds within the general formula (I), representative of it, and preparable by the processes of the invention.

TABLE I

Substituted xanthenone-4-acetic acids of Formula (I)

| No | $R_1$ | Mp(°C) | MW |
|---|---|---|---|
| 1 | H | 214-216 | 254.23 |
| 2 | 1-$CH_3$ | 206-209 | 268.26 |
| 3 | 1-$OCH_3$ | 223-227 | 284.26 |
| 4 | 1-Cl | 205-207 | 288.68 |
| 5 | 2-$CH_3$ | 243-245 | 268.26 |
| 6 | 2-$OCH_3$ | 229-231 | 284.26 |
| 7 | 2-Cl | 272-273 | 288.68 |
| 8 | 3-Cl | 214-218 | 288.68 |
| 9 | 5-$CH_3$ | 206-208 | 268.26 |
| 10 | 5-$CH_2CH_3$ | 210-211 | 282.27 |
| 11 | 5-Ph | 248-249 | 330.32 |
| 12 | 5-$OCH_3$ | 223-224 | 284.26 |
| 13 | 5-$OCH_2CH_3$ | 278-279 | 298.27 |
| 14 | 5-OH | 269-270 | 270.25 |
| 15 | 5-Cl | 238.5-239.5 | 288.68 |
| 16 | 5-$NO_2$ | 244-249 | 299.23 |
| 17 | 5-$NH_2$ | 266-267 | 269.25 |
| 18 | 5-$NHCOCH_3$ | 301-303 | 311.28 |
| 19 | 5-aza | 229-230 | 255.22 |
| 20 | 5-$CH_2COOH$ | 303-305 | 312.27 |
| 21 | 5-$CH_2CONHCH_3$ | 267-269 | 325.29 |
| 22 | 6-$CH_3$ | 224-225 | 268.26 |
| 23 | 6-$OCH_3$ | 205-207 | 284.26 |
| 24 | 6-OH | 303-305 | 270.25 |

(continued)

TABLE I (continued)

| No | $R_1$ | Mp(°C) | MW |
|---|---|---|---|
| 25 | 6-Cl | 248-249 | 288.68 |
| 26 | 7-$CH_3$ | 209-212 | 268.26 |
| 27 | 7-$OCH_3$ | 220-221 | 284.26 |
| 28 | 7-OH | 233-235 | 270.25 |
| 29 | 7-Cl | 198-199 | 288.68 |
| 30 | 7-$NO_2$ | 274-276 | 299.23 |
| 31 | 8-$CH_3$ | 198-201 | 268.26 |
| 32 | 8-$OCH_3$ | 223-225 | 284.26 |
| 33 | 8-Cl | 205-207 | 288.68 |
| 34 | 5,6-di$CH_3$ | 259-261 | 282.45 |
| 35 | 5,6-benz | 282-284 | 304.31 |
| 36 | 6,7-benz | 275-278 | 304.31 |

TABLE II

Elemental Analyses for the compounds of Table I

| No | Formula | Found C | Found H | Found N | Found Cl | Calculated C | Calculated H | Calculated N | Calculated Cl |
|---|---|---|---|---|---|---|---|---|---|
| 1 | $C_{15}H_{10}O_4$ | 71.0 | 3.9 | | | 70.9 | 4.0 | | |
| 2 | $C_{16}H_{12}O_4$ | 71.2 | 4.5 | | | 71.6 | 4.5 | | |
| 3 | $C_{16}H_{12}O_5$ | 67.4 | 4.4 | | | 67.6 | 4.3 | | |
| 4 | $C_{15}H_9ClO_4$ | 62.2 | 3.0 | | | 62.4 | 3.1 | | |
| 5 | $C_{16}H_{12}O_4$ | 71.6 | 4.8 | | | 71.6 | 4.5 | | |
| 6 | $C_{16}H_{12}O_5$ | 67.7 | 4.2 | | | 67.6 | 4.3 | | |
| 7 | $C_{15}H_9ClO_4$ | 62.2 | 2.9 | | 12.1 | 62.4 | 3.1 | | 12.3 |
| 8 | $C_{15}H_9ClO_4$ | 62.3 | 3.1 | | | 62.4 | 3.1 | | |
| 9 | $C_{16}H_{12}O_4$ | 71.5 | 4.4 | | | 71.6 | 4.5 | | |
| 10 | $C_{17}H_{14}O_4$ | 72.6 | 4.9 | | | 72.3 | 5.0 | | |
| 11 | $C_{21}H_{14}O_4$ | 76.7 | 4.1 | | | 76.4 | 4.3 | | |
| 12 | $C_{16}H_{12}O_5$ | 67.8 | 4.2 | | | 67.6 | 4.3 | | |
| 13 | $C_{17}H_{14}O_5$ | 68.5 | 4.8 | | | 68.4 | 4.7 | | |
| 14 | $C_{15}H_{10}O_5$ | 66.9 | 3.5 | | | 66.7 | 3.5 | | |
| 15 | $C_{15}H_9ClO_4$ | 62.3 | 2.9 | | 12.4 | 62.4 | 3.1 | | 12.3 |
| 16 | $C_{15}H_9NO_6$ | 59.8 | 2.9 | 4.8 | | 60.2 | 3.0 | 4.7 | |
| 17 | $C_{15}H_{11}NO_4$ | 66.6 | 4.0 | 5.2 | | 66.9 | 4.1 | 5.2 | |
| 18 | $C_{17}H_{13}NO_5$ | 65.3 | 4.2 | 4.6 | | 65.6 | 4.2 | 4.5 | |
| 19 | $C_{14}H_9NO_4$ | 65.7 | 3.4 | 5.5 | | 65.9 | 3.6 | 5.5 | |
| 20 | $C_{17}H_{12}O_6$ | 65.7 | 3.8 | | | 65.4 | 3.9 | | |
| 21 | $C_{18}H_{15}NO_5$ | 66.5 | 4.7 | 4.3 | | 66.5 | 4.7 | 4.3 | |
| 22 | $C_{16}H_{12}O_4$ | 71.7 | 4.4 | | | 71.6 | 4.5 | | |
| 23 | $C_{16}H_{12}O_5$ | 67.7 | 4.1 | | | 67.6 | 4.3 | | |

(continued)

Table II (continued)

Elemental Analyses for the compounds of Table I

| No | Formula | Found C | Found H | Found N | Found Cl | Calculated C | Calculated H | Calculated N | Calculated Cl |
|---|---|---|---|---|---|---|---|---|---|
| 24 | $C_{15}H_{10}O_5$ | 66.6 | 3.6 | | | 66.7 | 3.7 | | |
| 25 | $C_{15}H_9ClO_4$ | 62.4 | 2.9 | | 12.3 | 62.4 | 3.1 | | 12.3 |
| 26 | $C_{16}H_{12}O_4$ | 71.3 | 4.5 | | | 71.6 | 4.5 | | |
| 27 | $C_{16}H_{12}O_5$ | 67.5 | 4.2 | | | 67.6 | 4.3 | | |
| 28 | $C_{15}H_{10}O_5$ | 66.9 | 3.5 | | | 66.7 | 3.7 | | |
| 29 | $C_{15}H_9ClO_4$ | 62.4 | 3.1 | | 12.2 | 62.4 | 3.1 | | 12.3 |
| 30 | $C_{15}H_9NO_6$ | 60.3 | 3.0 | 4.5 | | 60.2 | 3.0 | 4.7 | |
| 31 | $C_{16}H_{12}O_4$ | 71.6 | 4.4 | | | 71.6 | 4.5 | | |
| 32 | $C_{16}H_{12}O_5$ | 67.5 | 4.2 | | | 67.6 | 4.3 | | |
| 33 | $C_{15}H_9ClO_4$ | 62.7 | 3.2 | | | 62.4 | 3.1 | | |
| 34 | $C_{17}H_{14}O_4$ | 72.5 | 5.3 | | | 72.3 | 5.0 | | |
| 35 | $C_{19}H_{12}O_4$ | 74.9 | 3.8 | | | 75.0 | 4.0 | | |
| 36 | $C_{19}H_{12}O_4$ | 74.8 | 3.7 | | | 75.0 | 4.0 | | |

The following Examples A-H illustrate the preparation of xanthenone-4-acetic acid and of compounds of general formula (I).

Example A.

Preparation of compound 1 of Table I by Method 1a of Scheme I.

A solution of 2-methylphenol (III : $R_3$ = H) (33g, 305 mmol) in MeOH (100 mL) was treated with Na (2.1g, 90 mmol), and excess MeOH was removed under reduced pressure. Diphenyliodonium-2-carboxylate (II : $R_1$ = H) (19.6g, 60 mmol) and cupric acetate (0.5g) were added and the mixture was stirred at 90 °C for 10h. The solution was diluted with 2N NaOH, filtered through celite and acidified with HCl. The mixture was then dissolved in 2N $K_2CO_3$ and extracted twice with EtOAc to remove excess 2-methylphenol. The aqueous layer was then poured into excess 2N HCl to give 2-(2-methylphenoxy)benzoic acid (IV : $R_1$ = $R_3$ = H) (8.85g, 64%), suitable for the next step. A sample crystallized from ligroin, mp 133-134 °C (Ber, 1905, 38, 2111 records mp 133.5 °C).

The above acid (8.85g, 39 mmol) was dissolved in polyphosphate ester (75g) and heated at 100 °C until all volatiles were removed, and for a further 30 min. The mixture was diluted with an equal volume of MeOH and basified with $Na_2CO_3$. Addition of water then precipitated 4-methylxanthenone (VI : $R_1$ = H, Z = $CH_3$), which was dried and crystallized from ligroin/ether to give needles (7.6g, 93%), mp 124-125 °C (Ber, 1905, 38, 2111 gives mp 126 °C).

A well-stirred mixture of the above 4-methylxanthenone (6.5g, 31 mmol), N-bromosuccinimide (5.5g, 31 mmol) and benzoyl peroxide (30 mg) in dry $CCl_4$ (250 mL) was heated under reflux with powerful illumination for 3h. The hot mixture was filtered, the filtrate was evaporated and the residue was crystallized from the minimum volume of boiling petroleum ether (ca. 1800 mL) to give 4-bromomethylxanthenone (VII : $R_1$ = H, Z = $CH_2Br$) (6.3g, 71%) as colourless needles, mp 191-192 °C. Anal. ($C_{14}H_9BrO_2$) C,H,N,Br.

The above 4-bromomethylxanthenone (5.77g, 20 mmol) was finely powdered and suspended in EtOH (150 mL). A hot solution of KCN (2.6g, 40 mmol) in water (25 mL) was added, and the mixture was heated

under reflux for 1 hour. A limited amount of hot water was then added to precipitate impurities which were removed by filtration. Further dilution with water then gave a crude product which was dried and crystallized from benzene-petroleum ether to give xanthenone-4-acetonitrile (VIII : $R_1$ = H, Z = $CH_2CN$) (2.9g, 62%). A sample crystallized from MeOH as prisms, mp 177-178 °C Anal ($C_{15}H_9NO_2$) C,H,N.

The above acetonitrile (2.0g) was dissolved in a mixture of AcOH (8 mL) and c.$H_2SO_4$ (8 mL), and heated under reflux for 90 min. Slow dilution with water gave a crystalline product, which was dissolved in warm aqueous $KHCO_3$. The solution was filtered and acidified with 2N HCl, and the resulting solid was crystallized from EtOH to give xanthenone-4-acetic acid (I ; $R_1$ = H) 1.6g, 74%), mp 214-216 °C.

The water-soluble sodium salt was crystallized from MeOH-EtOAc.

Example B

Preparation of compound 25 of Table I by Method 1b of Scheme I

A mixture of the sodium salts of 2,4-dichlorobenzoic acid (V : X = Cl, $R_1$ = 4-Cl) (27.7g 130 mmol) and 2-methylphenol (III : $R_3$ = H) (18.9g, 145 mmol) were dissolved in dry dioxan (300 mL). CuCl (1.3g, 13 mmol) and tris-[2-(2-methoxyethoxy)ethyl]amine (TDA-1) (4.2g, 13 mmol) were added, and the mixture was heated at reflux under $N_2$ for 8 hours. Excess solvent was evaporated under reduced pressure, and the residue was diluted with water and filtered. The filtrate was acidified with 2N HCl, and the resulting precipitate was collected, washed well with water and dried to yield 4-chloro-2-(2-methylphenoxy) benzoic acid (IV : $R_1$ = 4-Cl, $R_3$ = H) (27g, 79%), which was suitable for the next step. A sample was crystallized from aqueous MeOH as prisms, mp 158-159 °C. Anal. ($C_{14}H_{11}ClO_3$), C,H,Cl.

Similar reactions using appropriately substituted 2-halobenzoic acids (V and 2-methylphenols (III) gave the substituted 2-phenoxybenzoic acids (IV) listed in Table IIIwherein Z = $CH_3$..

The above crude acid (IV : $R_1$ = 4-Cl, $R_3$ = H) was cyclized with polyphosphate ester as in Example A to give a 90% yield of 6-chloro-4-methylxanthenone (VI : $R_1$ = 6-Cl, Z = $CH_3$), mp (MeOH) 145-146 °C. Anal. ($C_{14}H_9ClO_2$) C,H,N.

Similar cyclization of related substituted 2-phenoxybenzoic acids (IV, Z = $CH_3$) with polyphosphate ester gave the substituted methylxanthenones (VI) listed in Table IV.

The above compound (VI : $R_1$ = 6-Cl; Z = $CH_3$) was then treated with N-bromosuccinimide as in Example A to give 4-bromomethyl-6-chloroxanthenone (VII : $R_1$ = 6-Cl, Z = $CH_2Br$), mp (benzene/petroleum ether) 217-218 °C. Anal. ($C_{14}H_8BrClO_2$) C,H,N.

Similar reaction of methylxanthenones listed in Table IV gave the corresponding substituted bromomethylxanthenones listed in Table IV.

Reaction of the above compound (VII : $R_1$ = 6-Cl, Z = $CH_2Br$) with KCN as in Example A gave 6-chloroxanthenone-4-acetonitrile (VIII : $R_1$ = 6-Cl, Z = $CH_2CN$), mp (MeOH) 193-195 °C. Anal. ($C_{15}H_8ClNO_2$) C,H,N.

Similar reaction of bromomethylxanthenones listed in Table IV gave the corresponding substituted xanthenoneacetonitriles listed in Table IV.

The above acetonitrile (VIII : $R_1$ = 6-Cl, Z = $CH_2CN$) was hydrolysed in acid as in Example A to give 6-cloroxanthenone-4-acetic acid (I : $R_1$ = 6-Cl), (compound 25 of Table I) mp (MeOH) 248-249 °C. Anal. ($C_{15}H_9ClO_4$) C,H,Cl.

The sodium salt was crystallized from MeOH/EtOAc.

Similar reaction of xanthenone acetonitriles listed in Table IV gave the corresponding xanthenoneacetic acids listed in Table I.

This overall method was used to prepare compounds 4, 8, 9, 11, 12, 15, 19, 20, 23, 25, 27, 29, 33 and 35 of Table I.

TABLE III

Substituted 2-phenoxybenzoic acids (IV)

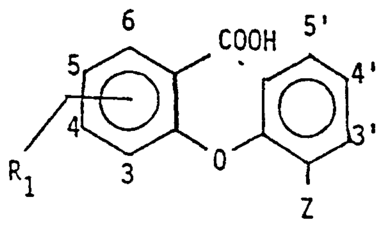

| $R_1$ | Z | Mp(°C) | Formula | Analysis |
|---|---|---|---|---|
| 5'-$CH_3$ | $CH_2COOH$ | 183-186 | $C_{16}H_{14}O_5$ | C,H |
| 5'-Cl | $CH_3$ | 116-118 | $C_{14}H_{11}ClO_3$ | C,H,Cl |
| 3'-Cl | $CH_3$ | 167-168 | literature[a] mp 168-169 | |
| 3'-$NO_2$ | $CH_3$ | 189-190 | literature[a] mp 187-190 | |
| 3-$OCH_3$ | $CH_2COOH$ | 186-187 | $C_{16}H_{14}O_6$ | C,H |
| 3-Cl | $CH_3$ | 125-126 | $C_{14}H_{11}ClO_3$ | C,H,Cl |
| 3-$NO_2$ | $CH_2COOH$ | 196-197 | $C_{15}H_{11}NO_7$ | C,H,N |
| 3-aza | $CH_3$ | 160-162 | $C_{13}H_{11}NO_3$ | C,H,N |
| 3-$CH_2CH_3$ | $CH_2COOH$ | 151-153 | $C_{17}H_{16}O_5$ | C,H |
| 4-$CH_3$ | $CH_2COOH$ | 209-211 | $C_{16}H_{14}O_5$ | C,H |
| 4-$OCH_3$ | $CH_3$ | 163.5-164.5 | $C_{15}H_{14}O_4$ | C,H |
| 5-$OCH_3$ | $CH_3$ | 132-133.5 | $C_{15}H_{14}O_4$ | C,H |
| 5-Cl | $CH_3$ | 125-126 | $C_{14}H_{11}ClO_3$ | C,H |
| 5-$NO_2$ | $CH_2COOH$ | 244-246 | $C_{15}H_{11}NO_7$ | C,H,N |
| 3,4-di$CH_3$ | $CH_2COOH$ | 240-242 | $C_{17}H_{16}O_5$ | C,H |
| 3,4-benz | $CH_2COOH$ | 197-200 | $C_{19}H_{14}O_5$ | C,H |

**Footnote for Table III**

[a] P. Dure, P. Valenti, G. Primafiore & L. Cima, Chim. Ther., 1973, 60.

TABLE IV

Substituted xanthenones

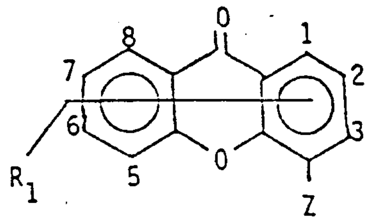

| $R_1$ | Z | Mp(°C) | Formula | Analysis |
|---|---|---|---|---|
| 1-Cl | $CH_3$ | 130-133 | $C_{14}H_9ClO_2$ | C,H,Cl |
| 1-Cl | $CH_2Br$ | 200-203 | $C_{14}H_8BrClO_2$ | C,H,Br |
| 1-Cl | $CH_2CN$ | 182-185 | $C_{15}H_8ClNO_2$ | C,H,N |
| 3-Cl | $CH_3$ | 164-166 | literature [a] mp 163-164 | |
| 3-Cl | $CH_2Br$ | 175-178 | literature [a] mp 186-188 | |
| 3-Cl | $CH_2CN$ | 214-217 | $C_{15}H_8ClNO_2$ | C,H,N |
| 5-$OCH_3$ | $CH_3$ | 205-206 | $C_{15}H_{12}O_3$ | C,H |
| 5-$OCH_3$ | $CH_2Br$ | 205-206 | $C_{15}H_{11}BrO_3$ | C,H,Br |
| 5-$OCH_3$ | $CH_2CN$ | 202-203 | $C_{16}H_{11}NO_3$ | C,H,N |
| 5-Cl | $CH_3$ | 176-176.5 | $C_{14}H_9ClO_2$ | C,H |
| 5-Cl | $CH_2Br$ | 181-182 | $C_{14}H_9BrClO_2$ | C,H,Br |
| 5-Cl | $CH_2CN$ | 162-163 | $C_{15}H_8ClNO_2$ | C,H,N |
| 5-aza | $CH_3$ | 153-155 | $C_{13}H_9NO_2$ | C,H,N |

**Footnote for Table IV**

[a] P. Dure, P. Valenti, G. Primafiore & L. Cima, Chim. Ther., 1973, 60.

(continued)

| $R_1$ | Z | Mp(°C) | Formula | Analysis |
|---|---|---|---|---|
| 5-aza | $CH_2Br$ | 214-216 | $C_{13}H_8BrNO_2$ | C,H,Br |
| 5-aza | $CH_2CN$ | 200-201 | $C_{14}H_8N_2O_2$ | C,H,N |
| 5-$CH_2Br$ | $CH_2Br$ | 257-258 | $C_{15}H_{10}BrO_2$ | C,H,Br |
| 5-$CH_2CN$ | $CH_2Br$ | 257-259 | $C_{17}H_{10}N_2O_2$ | C,H,N |
| 5-Ph | $CH_3$ | 140-141 | $C_{20}H_{14}O_2$ | C,H |
| 5-Ph | $CH_2Br$ | 184-185 | $C_{20}H_{13}BrO_2$ | C,H,Br |
| 5-Ph | $CH_2CN$ | 178-179 | $C_{21}H_{13}NO_2$ | C,H,N |
| 6-$OCH_3$ | $CH_3$ | 151-152 | $C_{15}H_{12}O_3$ | C,H |
| 6-$OCH_3$ | $CH_2Br$ | 179-180 | $C_{15}H_{11}BrO_3$ | C,H,Br |
| 6-$OCH_3$ | $CH_2CN$ | 203-204 | $C_{16}H_{11}NO_3$ | C,H,N |
| 6-Cl | $CH_3$ | 145-146 | $C_{14}H_9ClO_2$ | C,H, Cl |
| 6-Cl | $CH_2Br$ | 217-218 | $C_{14}H_8BrClO_2$ | C,H,Br |
| 6-Cl | $CH_2CN$ | 193-195 | $C_{15}H_8ClNO_2$ | C,H,N |
| 7-$OCH_3$ | $CH_3$ | 123-124 | $C_{15}H_{12}O_3$ | C,H |
| 7-$OCH_3$ | $CH_2Br$ | 183-185 | $C_{15}H_{11}BrO_3$ | C,H,Br |
| 7-$OCH_3$ | $CH_2CN$ | 203-205 | $C_{16}H_{11}NO_3$ | C,H,N |
| 7-Cl | $CH_3$ | 143-145 | $C_{14}H_9ClO_2$ | |
| 7-Cl | $CH_2Br$ | 200.5-201 | $C_{14}H_8BrClO_2$ | C,H,Br |
| 7-Cl | $CH_2CN$ | 198-199 | $C_{15}H_8ClNO_2$ | C,H,N |
| 5,6-benz | $CH_3$ | 230-231 | $C_{18}H_{12}O_2$ | C,H |
| 5,6-benz | $CH_2Br$ | 254-255 | $C_{18}H_{11}BrO_2$ | C,H,Br |
| 5,6-benz | $CH_2CN$ | 221.5-222 | $C_{19}H_{11}NO_2$ | C,H,N |

Example C

Preparation of compound 26 of Table I by Method 2a of Scheme II

2-[2-(Carboxymethyl)phenoxy]-5-methylbenzoic acid (XI: $R_1$ = 5-$CH_3$; $R_3$ = H)

A mixture of 10g(33 mmol) potassium 2-iodo-5-methylbenzoate, 7.8g (40 mmol, 1.2 equiv.) disodium 2-oxidophenylacetate, 0.4g (4 mmol) CuCl and 1.3g (4 mmol) tris[2-(2-methoxyethoxy)ethyl]amine (TDA-1) in 150ml dry dioxane was heated and stirred under reflux for 5 hours. The dioxane was removed under vacuum and the residue was dissolved in 100ml 0.1N NaOH solution. After filtration to remove insoluble copper salts the solution was acidified with dilute hydrochloric acid and extracted with ethyl acetate. The organic layer was then extracted with dilute aqueous ammonia and the resulting aqueous solution was added slowly with stirring to dilute hydrochloric acid. The resulting precipitate was collected and dried to give 8.72g (91% yield) of 2-[(2-carboxymethyl)phenoxy]-5-methylbenzoic acid (XI: $R_1$ = 5-$CH_3$, $R_3$ = H). M.p. (ethyl acetate) 226-227 °C. Anal. ($C_{16}H_{14}O_5$) C,H.

Ring closure of the above compound with 90% $H_2SO_4$ gave an 87% yield of 7-methyl-xanthone-4-acetic acid (compound 26 of Table I)

Similar reactions using appropriately substituted 2-halobenzoic acids (V) and hydroxyphenylacetic acids (X) gave the phenoxyphenylacetic acids (IV) listed in Table III wherein Z = $CH_2COOH$ (i.e. compounds of formula (XI)). Similar ring closure gave the corresponding xanthenone-4-acetic acids.

This method was used to prepare compounds 2, 10, 12, 13, 16, 22, 30, 31, 34, 35 and 36 of Table I.

Example D

Preparation of compound 5 of Table I by the method of Scheme III

(i) 4 Allyl-2-methylxanthenone (XIX; $R_1$ = H; $R_3$ = 2-$CH_3$)

A mixture of 13.6g (70 mmol) potassium 2-chlorobenzoate, 14.3g (84 mmol, 1.2 equiv.) sodium 2-allyl-4-mekthylphenoxide, 0.8g (8 mmol) CuCl and 2.6g (8 mmol) tris[2-(2-methoxyethoxy]amine (TDA-1) in 200ml anisole was heated and stirred under reflux for 3 hours. The anisole was removed under vacuum and the residue was extracted with dilute aqueous ammonia. After filtration to remove insoluble inorganics the aqueous solution was washed twice with ethyl acetate to remove remaining traces of anisole and excess 2-allyl-4-methyl-phenol). Acidification with conc. hydrochloric acid then gave an oil which was extracted into ethyl acetate. The organic layer was dried ($N_2SO_4$) and the ethyl acetate was removed to give 10.6 g (47% yield) of crude 2-(2-allyl-4-methylphenoxy)benzoic acid (XVIII; $R_1$ = H; $R_3$ = 4′-$CH_3$), as an oil. The oil was dissolved in 50ml of a chloroform solution of polyphosphate ether (PPE) and heated on a waterbath with the solvent being allowed to boil off. After 30min the residue was basified with dilute sodium hydroxide solution and the oil product was extracted into petroleum ether. After being dried ($Na_2SO_4$) the solvent was removed to give 6.5g (66% yield) of 4-allyl-2-methylxanthenone (XIX: $R_1$ = H; $R_3$ = 2-$CH_3$; m.p. (methanol) 97-98 °C. Anal. ($C_{17}H_{14}O_2$) C,H.

Similar reactions using appropriately substituted 2-allyl-phenoxides (XVII) gave the following compounds of formula (XVIII)

| $R_3$ | Z | Mp(°C) | Formula | Analysis |
|---|---|---|---|---|
| 4'-$OCH_3$ | $CH_2CH=CH_2$ | 168-170 | $C_{17}H_{16}O_4$ | C,H |
| 4'-Cl | $CH_2CH=CH_2$ | 133-135 | $C_{16}H_{13}ClO_3$ | C,H |

and then the following compounds of formula (XIX)

| $R_1$ | Z | Mp(°C) | Formula | Analysis |
|---|---|---|---|---|
| 2-$OCH_3$ | $CH_2CH=CH_2$ | 109-112 | $C_{17}H_{14}O_3$ | C,H |
| 2-Cl | $CH_2CH=CH_2$ | 110-111 | $C_{16}H_{11}ClO_2$ | C,H |

(ii) 2-Methylxanthenone-4-acetic acid

A solution of 5g (20 mmol) 4-allyl-2-methylxanthenone in a mixture of 75ml acetic acid, 75ml acetone and 50ml water was cooled to below 5 °C and 15.8g (5 equiv.) $KMnO_4$ was added in portions over 6 hours. After being stirred for a further 1 hour the mixture was poured into 1l of water and $Na_2S_2OS_n$ to remove $MnO_2$. The remaining solid was collected by filtration and dissolved in dilute aqueous ammonia solution. After treatment with charcoal and filtration through celite the clear solution was acidified with conc. hydrochloric acid to give 3.20g (60% yield) of 2-methylxanthenone-4-acetic acid (compound 5 of Table I); m.p. (ethanol) 243-245 °C. Anal. ($C_{16}H_{12}O_4$) C,H.

Similar reaction of the other compounds of formula (XIX) listed above gave compounds 6 and 7 of Table I.

Example E

Preparation of compound 11 of Table I by the method of Scheme IV

A mixture of 2-hydroxy-3-methylbenzoic acid (60.8g. 0.4 mol) 2-hydroxybiphenyl (68g, 0.4 mol) and polyphosphate ester (Pollman and Schramm, Biochim. Biophys. Acta., 1964, 80, 1) (180mL; used without solvent removal) was heated on a water bath with occasional swirling for 3 hours. Solvent still remaining after this time was removed in vacuo; the residue was poured on to crushed ice; excess powdered $NaHCO_3$ was added and then the mixture was stood at room temperature for 12 hours. The whole was extracted with $CH_2Cl_2$ (500mL), filtered and the extract was washed with aqueous $NaHCO_3$ and then dried and evaporated. Extraction of the resulting oil with hot petroleum ether (b.p. 40-60 °C) followed by solvent evaporation gave crude biphenyl ester (89g, 73% yield) of sufficient purity (c.a. 85%) for use in the next stage.

Crude 2-biphenyl 2-hydroxy-3-methylbenzoate (78g, c.a. 85% pure) was heated in a flask connected to a short distillation pathway to 280-300 °C when a vigorous exotherm occurred resulting in rapid distillation of the pyrolysate. The highest bp fraction (340-370 °C c.a. 26g) was dissolved in boiling EtOH (300mL), treated with 5N aqueous NaOH (60mL), heated under reflux for 5 min and then the resulting solution was diluted with water. The solid which separated on cooling was collected, washed with water and recrystallised twice from petroleum ether (bp 80-100 °C) (charcoal) to give pure 4-methyl-5-phenylxanthenone (XXIII: $R_1$ = Ph) 8.8g, 12% yield based on crude starting material) as colourless needles, m.p. 140-141 °C. Anal.-($C_{20}H_{14}O_2$) C,H.

This compound was then elaborated by the method outlined in Example A to give compound 11 of Table I.

Example F

Preparation of compound of 9 of Table I

4-Bromomethyl-5-methylxanthenone

Powdered N-bromosuccinimide (5.0g, 0.028 mol) was added to warm solution of 4,5-dimethylxanthenone (prepared by the method of Schopff, Ber 1892, 25, 3642) in $CCl_4$ (170 mL) containing benzoyl peroxide (40 mg), and the mixture was stirred at reflux temperature under UV irradiation (illumination) for 3 hours. Removal of the solvent under reduced pressure gave a residue which was extracted with $CHCl_3$. The $CHCl_3$ layer was washed with cold 1N aqueous NaOH and water (twice), then dried and evaporated to give the crude product, contaminated with starting material and the 4,5-dibromomethylxanthenone. Repeated crystallization from benzene-petroleum ether gave pure material as colourless needles (1.96g, 29%), m.p. 171-172 °C. Anal. ($C_{15}H_{11}BrO_2$) C,H,Br.

5-Methylxanthenone-4-acetonitrile

Powdered 4-bromomethylxanthenone (6.06g, 0.02 mol) was added to a hot solution of NaCN (2.6g, 0.04 mol) in water (30 mL) and EtOH (180 mL), and the mixture was stirred under reflux until homogenous and for a further 10 min. The hot solution was filtered and concentrated until separation of the product commenced. After cooling, the solid was collected and crystallized from EtOH to give the pure acetonitrile as prisms (3.1g, 62%), mp 177-178 °C. Anal. ($C_{16}H_{11}NO_2$) C,H,N.

5-Methylxanthenone-4-acetic acid (compound 9 of Table I)

A mixture of the above acetonitrile (2.1g), AcOH (8 mL), water (8 mL) and conc. $H_2SO_4$ (8 mL) was heated under reflux for 2 hours, then cooled and diluted with twice the volume of water. The resulting precipitate was collected, washed well with water, and extracted with warm dilute aqueous $KHCO_3$. The extract was clarified by filtration and acidified to give the desired product (2.08g, 92%). Further crystallization from aqueous EtOH gave white prisms, mp 206-208 °C. Anal. ($C_{16}H_{12}O_4$) C,H.
The sodium salt was crystallized from MeOH/EtOAc as prisms.

Example G

Preparation of compound 2 of Table I by methods 2a and 2b of Scheme II

4-Methyl-2-phenylmethoxybenzoic acid (XII: $R_3$ = 4-$CH_3$)

A two-phase mixture of 45.6g (0.3 mol) 4-methylsalicylic acid, NaOH (36g, 0.9 mol), benzyl bromide (154g, 0.9 mol) and tetrabutylammonium bromide (10g, 30 mmol) in water (200ml) and $CH_2Cl_2$ (200ml) was stirred at room temperature for 3 hours. The layers were separated and the $CH_2Cl_2$ was removed from the organic fraction. The residue was dissolved in a mixture of ethanol (250ml) and 2N NaOH (50ml), and the mixture was heated under reflux for 30 min. The ethanol was removed under vacuum and the residue was diluted with water and washed with ethyl acetate. The aqueous layer was separated and acidified with dilute HCl to give a precipitate of 4-methyl-2-phenylmethoxybenzoic acid (65.3g, 90%) m.p. (MeOH aq) 105-107°C. Anal. ($C_{15}H_{14}O_3$) C,H,O.

4-Methyl-2-phenylmethoxybenzenemethanol (XIII: $R_3$ = 4-$CH_3$, Z = OH)

A solution of 4-methyl-2-phenylmethoxy-benzoic acid (30.3g, 0.125 mol) in 100ml thionyl chloride containing 3 drops DMF was heated under reflux for 30 min. The $SOCl_2$ was removed under vacuum and the residual oil was diluted with 100ml dry benzene. The solvent was again removed under vacuum to remove remaining traces of $SOCl_2$ and the crude acid chloride product was added slowly to a solution of $NaBH_4$ (10g) in dry diglyme (200ml) at 10-20°C. The resulting mixture was stirred for 30min at room temperature and the solvent was removed under vacuum by heating in an oilbath. Water (100ml) was added slowly to the white solid and then acetic acid (10ml) was added to ensure complete decomposition of the excess borohydride. The mixture was basified with conc. ammonia and extracted with ethyl acetate to give 4-methyl-2-phenylmethoxybenzenemethanol (26.6g, 93% crude yield) as an oil.

1-Bromomethyl-4-methyl-2-phenylmethoxybenzene (XIV: $R_3$ = 4-$CH_3$, Z = Br)

A solution of crude 4-methyl-2-phenylmethoxy-benzenemethanol (20g, 87.6 mmol) in dry benzene (100ml) was treated with 9.1ml (96 mmol) $PBr_3$ at room temperature and after being stirred for 10min the mixture was treated with 50ml 2N NaOH solution. The organic layer was separated and dried ($Na_2SO_4$) to give 1-bromomethyl-4-methyl-2-phenylmethoxybenzene (23.7g, 93% crude yield) as an oil.

(4-Methyl-2-phenylmethoxyphenyl)acetronitrile (XV: $R_3$ = 4-$CH_3$, Z = CN)

A two-phase mixture of crude 1-bromomethyl-4-methyl-2-phenylmethoxybenzene (21.84g, 75 mmol), NaCN (11.0g, 0.22 mol), tetrabutylammonium bromide (2.4g, 7.5 mmol), water (25ml) and $CH_2Cl_2$ (50ml) was stirred at room temperature for 1 hour and the layers were separated. The organic layer was washed well with water to remove tetrabutylammonium salts and after drying ($CaCl_2$) the solvent was removed to give (4-methyl-2-phenylmethoxyphenyl)acetonitrile (17.8g, 100% crude yield) as an oil.

(2-Hydroxy-4-methylphenyl)acetic acid (X: $R_3$ = 4-$CH_3$

A solution of crude 2-(4-methyl-2-phenylmethoxyphenyl)acetonitrile (15g, 63 mmol) in ethanol (200ml) and water (50ml) containing 10g (0.25 mol) NaOH was heated under reflux overnight, and the ethanol was removed under vacuum. The residue was diluted with water and washed with benzene. The aqueous layer was then acidified with dilute HCl to give an oil which was extracted into ethyl acetate to give (4-methyl-2-phenylmethoxyphenyl)acetic acid (14.2g, 88%) as an oil which solidified on standing. A solution of this crude (4-methyl-2-phenylmethoxyphenyl)acetic acid in ethanol was hydrogenated over palladium on charcoal to give (2-hydroxy-4-methylphenyl)acetic acid. Anal ($C_9H_{10}O_3$) C,H.

2-[2-(Carboxymethyl)-5-methylphenoxy]benzoic acid (XI: $R_1$ = H; $R_3$ = 5′-$CH_3$)

A mixture of potassium 2-chlorobenzoate (9.2g, 47 mol), disodium 2-(4-methyl-2-oxide-phenyl)acetate (6.2g, 29 mmol) (prepared from 4.9g (2-hydroxy-4-methylphenyl) acetic acid and sodium hydroxide in methanol), CuCl (1g, 10 mmol) and tris[2-(2-methoxyethoxy)ethyl]amine (TDA-1) (3.2g, 10 mmol) in dry dioxane (200ml) was refluxed and stirred for 5 hours and the dioxane was removed under vacuum. The

residue was dissolved in dilute ammonia solution and the solution was filtered. Acidification with conc. HCl gave the crude product which was extracted into ethyl acetate and washed with water. The product was extracted into dilute ammonia solution again and the resulting solution was poured slowly into dilute HCl to give a precipitate of [2-(carboxymethyl)-5-methylphenoxy]benzoic acid (4.70g, 55%), m.p. (ethyl acetate) 183-186 °C. Anal ($C_{16}H_{14}O_5$).

Compound 2 of Table I

[2-(carboxymethyl)-5-methylphenoxy]benzoic acid (4g, 14 mmol) was dissolved in a freshly prepared solution of conc. $H_2SO_4$ (80ml) and water (20ml) and after a further 5 min at >80 °C the mixture was poured into water. The solid was collected by filtration and washed with water and 50% aqueous methanol. Recrystallization from ethanol gave 1-methylxanthenone-4-acetic acid (3g, 80%), m.p. 206-209 °C. Anal ($C_{16}H_{12}O_4$) C,H.

Example H

Preparation of compound 28 of Table I

A suspension of 7-methoxyxanthenone-4-acetic acid (compound 27 of Table I) (2.0g) in 48% w/w HBr (30mL) was heated under reflux for 3 hours. The precipitate which formed on cooling was collected, washed with HBr and dissolved in aqueous $KHCO_3$. This solution was filtered and acidified with 2N HCl to give the crude product. Crystallization from MeOH gave pure compound 28 (1.51g, 79%) as colourless needles, m.p. 232-234. Anal ($C_{15}H_{10}O_4$) C,H.

Compounds 14 and 24 of Table I were prepared by similar methods from compounds 12 and 23, respectively, of Table I.

Xanthenone-4-acetic acid and the compounds of general formula (I) have antitumour activity, as shown, for example, by the data given in Table V.

The cytotoxic activity of the compounds was measured in four different ways.

(1) Inhibition of growth of cultured cells

Mouse L1210 lymphocytic leukaemia cells were grown in RPM1 1640 medium supplemented with 10% foetal bovine serum, 50um 2-mercaptoethanol, 100 units/ml penicillin and 100ug/ml streptomycin as previously described (Baguley and Nash, Eur.J. Cancer, 17, 671-679, 1981). The toxic effect of xanthoneacetic acid derivatives was measured by exposure of the cells to the drug over a period of 70 hours. The $IC_{50}$ value is the drug concentration which reduces the cell number by 50% with respect to untreated cultures, and the following values (in $\mu$M) were determined:

Compound 1, >29$\mu$M; 9, 16$\mu$M; 25, 21$\mu$M; 30, 29$\mu$M.

Data for the other three assays is recorded in Table V.

(2) Activity against the P388 leukemia in mice

Mouse P388 leukemia cells were obtained as frozen cell stock from Mason Research Inc., USA, and were passaged intraperitoneally in DBA/25 mice of either sex, according to standard methods (Cancer Chemotherapy Reports, 1972, 3,9). Groups of six mice (F1 hybrids of DBA(2J male x C57 BL/6J female) were injected intraperitoneally with $10^6$ tumour cells on day 0. Antitumour activity was determined by published methods (Eur. J. Cancer, 19, 1607, 1983).

OD is the optimal drug dose (in milligrams per kilogram) administered intraperitoneally as a solution in 0.1 ml of 30% v/v ethyl alcohol/water on days 1, 5 and after tumour inoculation, the drug being given as a soluble basis addition salt.

$ILS_{max}$ is the percentage increase in lifespan of treated animals over that of control animals injected with tumour alone. The average survival of control mice was 11 days. ILS values greater than 20% are considered statistically significant.

NA implies no activity

(3) Induction of haemorrhagic necrosis in Colon 38 tumours in mice

Colon 38 tumour was obtained from Mason Research Inc. and passaged subcutaneously (sc) in C57BL/6J mice. Fragments (1 $mm^3$ of an advanced sc tumour were inoculated sc into BDF1 (DBA/2J male x C57BL/6J female) mice and allowed to grow to a diameter of approximately 10mM. Compounds were dissolved in $H_20$ and injected intraperitoneally. After 24 hours the tumour was removed surgically and fixed in 10% formaldehyde solution. Sections were made and stained with haematoxylin and eosin according to standard histological methods. Sections were examined by a histopathologist and scored as follows:

- : No evidence of toxic effects in comparison to untreated tumours (some areas of necrosis are usually observed in such tumours).
+ : Evidence of cytopathological changes across the whole section.
+ + : Extensive haemorrhagic necrosis across the whole section.

(4) Induction of natural killer cell activity

BDF, hybrid mice (CS7 BL/6J x DBA/2J) were injected with drug intraperitoneally at the maximum tolerated dose. After 12 hours, spleen cells were collected and assayed for natural killer cell (NK) actity using the $^{51}$Cr assay as published (Eur. J. Cancer Clin. Oncol., 1987, 23, 1047), and the results assessed as follows:

NA not active
+ equal activity to flavoneacetic acid
+ + higher activity than flavoneacetic acid
- suppression of NK activity

Selected compounds were assayed for their ability to induce a delay in the growth of subcutaneous colon 38 tumours in mice, and these results are given in Table VI.

The colon 38 tumour was grown as above. Compounds were administered intraperitoneally in aqueous solution. Tumour diameters (major and minor axes) were measured twice weekly thereafter and calculated tumour volumes were compared to those of untreated control mice.

Under similar conditions the clinical agents, 5-fluorouracil (65 mg/Kg every 4 days x 3) and cyclophosphamide (220 mg/Kg, single dose) provided no complete regressions and mean tumour growth delays of 10 and 4 days respectively.

TABLE V

Biological activity of the compounds of Table I

| No | P388 in vivo OD | P388 in vivo ILS | colon 38 (histology) OD | colon 38 (histology) assessment | dose | NK activity assessment |
|---|---|---|---|---|---|---|
| 1 | | | 220 | ++ | 220 | + |
| 2 | | | 220 | + | | |
| 3 | | | 330 | ± | | |
| 4 | | | 330 | ± | | |
| 5 | | | 220 | ++ | 330 | - |
| 6 | | | 750 | + | 330 | - |
| 7 | | | 330 | ± | | |
| 8 | | | 220 | + | | |
| 9 | 45 | 30 | 45 | ++ | 45 | ++ |
| 10 | | | 150 | ++ | | |
| 11 | | | 220 | - | | |
| 12 | | | 150 | + | | |
| 13 | | | 330 | ++ | | |
| 14 | | | 330 | + | | |
| 15 | 65 | 34 | 100 | ++ | 100 | ++ |
| 16 | | | 150 | + | | |
| 17 | | | 500 | - | | |
| 18 | | | 500 | - | | |
| 19 | | | 500 | - | | |
| 20 | 150 | NA | 330 | - | 500 | NA |
| 21 | 225 | NA | 330 | - | | |
| 22 | | | 220 | ++ | 220 | + |
| 23 | 225 | NA | 150 | ++ | 150 | ++ |

(continued)

TABLE V (continued)

| No | P388 in vivo OD | P388 in vivo ILS | colon 38 (histology) OD | colon 38 (histology) assessment | NK activity dose | NK activity assessment |
|---|---|---|---|---|---|---|
| 24 | | | 500 | - | | |
| 25 | 150 | NA | 150 | ++ | 150 | + |
| 26 | | | 500 | + | | |
| 27 | 150 | NA | 330 | - | 330 | NA |
| 28 | | | 500 | ± | | |
| 29 | 225 | NA | 500 | - | 500 | NA |
| 30 | 225 | NA | 330 | - | 330 | NA |
| 31 | | | 330 | - | | |
| 32 | | | 330 | + | | |
| 33 | | | 750 | ± | | |
| 34 | | | 30 | ++ | | |
| 35 | | | 100 | ++ | | |
| 36 | | | 150 | ± | | |

TABLE VI

Growth delay of subcutaneous colon 38 tumours *in vivo*

| compound | dose (mg/Kg) | schedule | growth delay (days) |
|---|---|---|---|
| 1 | 220 | 2 doses, 7 days apart | 11 |
| 9 | 45 | 2 doses, 7 days apart | 13 |
| 15 | 100 | single dose | 5.2 |
| 25 | 100 | single dose | 0 |

The results shown in Table V indicate that xanthenone-4-acetic acid and the compounds of formula (I) are useful as antitumour drugs, particularly against solid tumours, and also have potent immunostimulatory properties.

The present invention therefore also provides a compound of formula (I) or a pharmaceutically acceptable salt thereof, for use as a medicament, especially in the treatment of tumours and in particular cancers.

The present invention further provides the use of xanthenone-4-acetic acid or a compound of formula (I) or a pharmaceutically acceptable salt thereof for the manufacture of a medicament or use in the treatment of tumours.

The present invention further provides pharmaceutical compositions having antitumour and/or immunostimulatory properties and comprising at least one compound of general formula (I) or a pharmaceutically acceptable salt thereof and one or more pharmaceutically acceptable carriers or diluents.

The active compounds may be orally administered, for example, with an inert diluent or with an assimilable edible carrier, or they may be enclosed in hard or soft shell gelatin capsules, or they may be compressed into tablets, or they may be incorporated directly with the food of the diet. For oral therapeutic administration, the active compounds may be incorporated with excipients and used in the form of ingestible tablets, buccal tablets, troches, capsules, elixirs, suspensions, syrups, wafers and the like. Generally such compositions and preparations should contain at least 0.1% of active compound. The percentage in the compositions and preparations may, of course, be varied and may conveniently be from about 2 to about 60% of the weight of the unit. The amount of active compound in such therapeutically useful compositions is such that a suitable dosage will be obtained. Preferred compositions or preparations according to the present invention are prepared so that an oral dosage unit form contains from about 5 to about 200 milligrams of active compound.

The tablets, troches, pills, capsules and the like may also contain, for example, one or more of the following: a binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavouring agent such as peppermint, oil of wintergreen or cherry flavouring. When the dosage unit form is a capsule, it may contain, for example, in addition to materials of the above type, a liquid carrier. Various other materials may be present as coatings or to otherwise modify the physical form of the dosage unit. For instance, tablets, pills or capsules may be coated with shellac, sugar or both. A syrup or elixir may contain the active compound, sucrose as a sweetening agent, methyl and propylparabens as preservatives, a dye and flavouring such as cherry or orange flavour. Of course any material used in preparing any dosage unit form should be pharmaceutically pure and substantially non-toxic in the amounts employed. In addition, the active compounds may be incorporated into sustained-release preparations and formulations.

The active compounds may also be administered parenterally or intraperitoneally. Solutions of the active compound as a free base or pharmaceutically acceptable salt can be prepared in water suitably mixed with a surfactant such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof and vegetable oils. The proper fluidity can be maintained, for example, by the use of a coating such as lecithin, by the maintenance of the required particle size in the case of dispersions and by the use of surfactants. The prevention of the action of microorganisms can be brought about by various antibacterial and antifungal agents, for example, parabens, chlorobutanol, phenol, sorbic acid, thimerosal and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions may be prepared by incorporating the active compound in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from a previously sterile-filtered solution thereof.

As used herein, "pharmaceutically acceptable carrier" includes any and all suitable solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into

the compositions.

It is especially advantageous to formulate parenteral compositions in dosage unit form for ease of administration and uniformity of dosage. Dosage unit form as used herein refers to physically discrete units suitable as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired.

The principal active ingredient is compounded for convenient and effective administration in effective amounts with a suitable pharmaceutically-acceptable carrier in dosage unit form as hereinbefore disclosed. A unit dosage form can, for example, contain the principal active compound in amounts ranging from about 0.1 to about 400 mg, with from about 1 to about 30 mg being preferred. Expressed in proportions, the active compound is generally present in from about 0.1 to about 400 mg/ml of carrier. In the case of compositions containing supplementary active ingredients, the dosages are determined by reference to the usual dose and manner of administration of the said ingredients.

**Claims**

**Claims for the following Contracting States : AT, BE, CH, DE, FR, GB, IT, LI, LU, NL, SE**

**1.** A compound represented by the general formula (I)

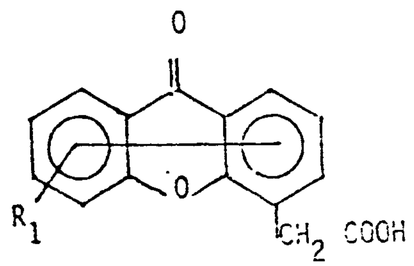

(I)

where $R_1$ represents up to two of the groups ($C_1$-$C_5$)-alkyl, halogen, phenyl, $CF_3$, CN, $NO_2$, $NH_2$, $CH_2COOH$, $OR_2$, OH, $NHCOR_2$, $NHSO_2R_2$, $SR_2$, $SO_2R_2$, $CH_2CONHR_2$ or $NHR_2$ (where $R_2$ is ($C_1$-$C_5$)-alkyl optionally substituted with hydroxy, amino or methoxy functions), at any of the positions 1-8 which are available, $R_1$ may also represent the substitution of an aza (-N=) group for one or two of the methine (-CH=) groups in the carbocyclic rings and two of $R_1$ on any two available adjacent positions may also represent the grouping -CH=CH-CH=CH- to form an additional fused benzene ring; and basic addition salts thereof.

**2.** A compound according to claim 1 where $R_1$ is 2-$CH_3$.

**3.** A compound according to claim 1 where $R_1$ is 2-$OCH_3$.

**4.** A compound according to claim 1 where $R_1$ is 5-$CH_3$.

**5.** A compound according to claim 1 where $R_1$ is 5-$OCH_3$

**6.** A compound according to claim 1 where $R_1$ is 5-Cl.

**7.** A compound according to claim 1 where $R_1$ is 6-$CH_3$.

**8.** A compound according to claim 1 where $R_1$ is 6-$OCH_3$.

**9.** A compound according to claim 1 where $R_1$ is 6-Cl.

**10.** A compound according to claim 1 where $R_1$ is 5,6-di$CH_3$.

**11.** A compound according to claim 1 where $R_1$ is the grouping -CH = CH-CH = CH- attached at the 5 and 6 positions to form an additional fused ring.

**12.** A process for the preparation of a compound represented by the general formula (I) as defined in claim 1, or a basic addition salt thereof, which process comprises cyclodehydrating a substituted phenoxybenzoic acid of the general formula (IV)

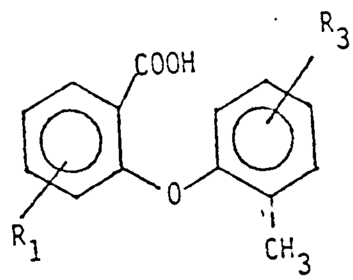

(IV)

where $R_1$ is hydrogen or is $R_1$ as defined in claim 1 and $R_3$ is as defined for $R_1$, not more than one of $R_1$ and $R_3$ being hydrogen; brominating the obtained 4-methylxanthenone of the general formula (VI)

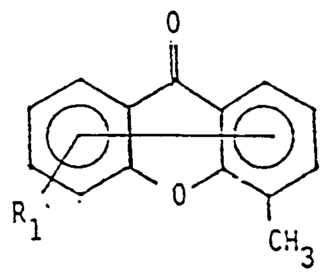

(VI)

reacting the obtained 4-bromomethylxanthenone of the general formula (VII)

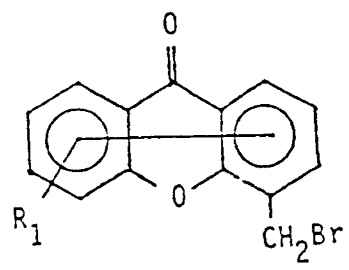

(VII)

with an inorganic cyanide, and hydrolysing the obtained xanthenone-4-acetonitrile of the general formula (VIII)

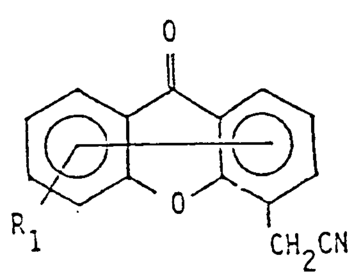

(VIII)

to the corresponding compound of formula (I) and, if desired converting the compound of formula (I) to a basic addition salt thereof.

**13.** A process for the preparation of a compound represented by the general formula (I) as defined in claim 1, or a basic addition salt thereof, which process comprises cyclodehydrating a substituted phenoxyphenylacetic acid of the general formula (XI)

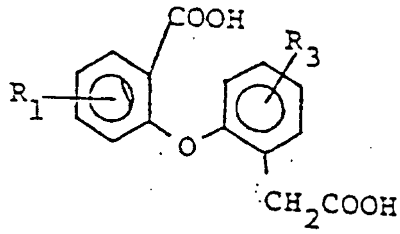

(XI)

where $R_1$ is hydrogen or is $R_1$ as defined in claim 1 and $R_3$ is as defined for $R_1$, not more than one of $R_1$ and $R_3$ being hydrogen, to the corresponding compound of formula (I) and, if desired, converting the compound of formula (I) into a basic addition salt thereof.

**14.** A process for the preparation of a compound represented by the general formula (I) as defined in claim 1, or a basic addition salt thereof, which process comprises cyclodehydrating a compound of the general formula (XVIII)

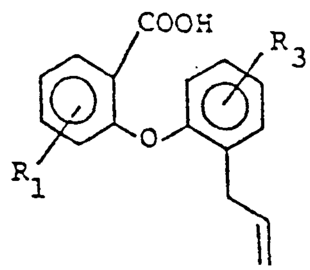

(XVIII)

where $R_1$ is hydrogen or is $R_1$ as defined in claim 1 and $R_3$ is as defined for $R_1$, not more than one of $R_1$ and $R_3$ being hydrogen; oxidising the obtained 4-allylxanthenone of the general formula (XIX)

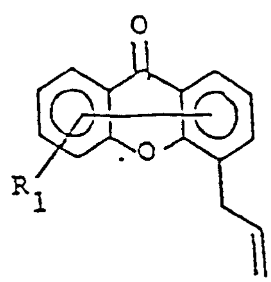

(XIX)

to the corresponding compound of formula (I) and, if desired, converting the compound of formula (I) into a basic addition salt thereof.

**15.** A process for the preparation of a compound of the general formula (I) as defined in claim 1, wherein $R_1$ is in the 5-position, or a basic addition salt thereof, which process comprises subjecting a compound of the general formula (XXII)

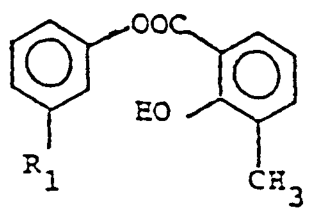

(XXII)

wherein $R_1$ is as defined in claim 1, to controlled pyrolysis, brominating the obtained 4-methylxanthenone of the general formula (XXIII)

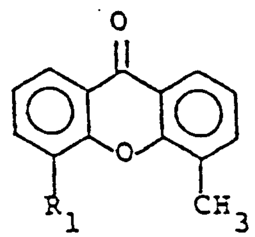

(XXIII)

reacting the obtained 4-bromomethylxanthenone with an inorganic cyanide; and hydrolysing the obtained xanthenone-4-acetonitrile to the corresponding compound of formula (I) and, if desired, converting the compound of formula (I) to a basic addition salt thereof.

**16.** A pharmaceutical composition having antitumour activity which comprises at least one compound of the general formula (I) defined in claim 1, or a pharmaceutically-acceptable basic addition salt thereof and one or more pharmaceutically acceptable carriers or diluents.

**17.** A pharmaceutical composition having antitumour activity which comprises a compound according to any one of claims 2 to 11, or a pharmaceutically-acceptable basic addition salt thereof, or one or more pharmaceutically-acceptable carriers or diluents.

**18.** A compound of the general formula (I) defined in claim 1, or a pharmaceutically acceptable basic addition salt thereof, for use in the treatment of tumours.

**19.** A compound according to any one of claims 2 to 11, or a pharmaceutically acceptable basic addition salt thereof, for use in the treatment of tumours.

**20.** The use of xanthenone-4-acetic acid or of a compound of the general formula (I) as claimed in any one of claims 1 to 11, or a pharmaceutically acceptable basic addition salt thereof, for the manufacture of a medicament for use in the treatment of tumours.

**Claims for the following Contracting States : ES, GR**

**1.** A process for the preparation of a compound represented by the general formula (I)

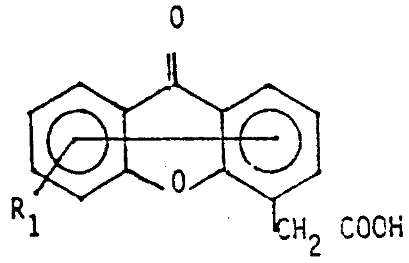

(I)

where $R_1$ represents up to two of the groups ($C_1$-$C_5$)-alkyl, halogen, phenyl, $CF_3$, CN, $NO_2$, $NH_2$, $CH_2COOH$, $OR_2$, OH, $NHCOR_2$, $NHSO_2R_2$, $SR_2$, $SO_2R_2$, $CH_2CONHR_2$ or $NHR_2$ (where $R_2$ is ($C_1$-$C_5$)-alkyl optionally substituted with hydroxy, amino or methoxy functions), at any of the positions 1-8 which are available, $R_1$ may also represent the substitution of an aza (-N=) group for one or two of the methine (-CH=) groups in the carbocyclic rings and two of $R_1$ on any two available adjacent positions may also represent the grouping -CH=CH-CH=CH- to form an additional fused benzene ring;
or a basic addition salt thereof, which process comprises cyclodehydrating a substituted phenoxybenzoic acid of the general formula (IV)

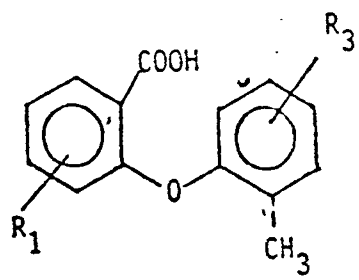

(IV)

where $R_1$ is hydrogen or is $R_1$ as defined above and $R_3$ is as defined for $R_1$, not more than one of $R_1$ and $R_3$ being hydrogen; brominating the obtained 4-methylxanthenone of the general formula (VI)

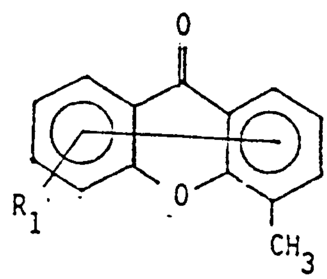

(VI)

reacting the obtained 4-bromomethylzanthenone of the general formula (VII)

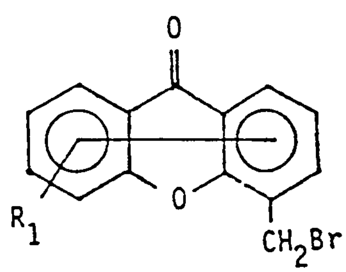

(VII)

with an inorganic cyanide, and hydrolysing the obtained xanthenone-4-acetonitrile of the general formula (VIII)

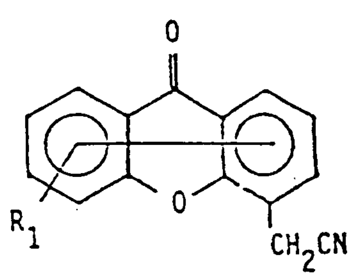

(VIII)

to the corresponding compound of formula (I) and, if desired converting the compound of formula (I) to a basic addition salt thereof.

2. A process for the preparation of a compound represented by the general formula (I) as defined in claim 1, or a basic addition salt thereof, which process comprises cyclodehydrating a substituted phenoxyphenylacetic acid of the general formula (XI)

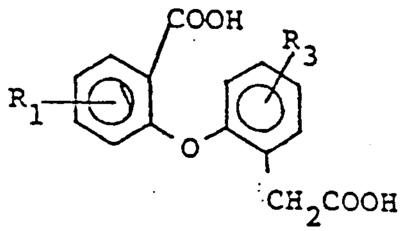

(XI)

where $R_1$ is hydrogen or is $R_1$ as defined in claim 1 and $R_3$ is as defined for $R_1$, not more than one of $R_1$ and $R_3$ being hydrogen, to the corresponding compound of formula (I) and, if desired, converting the compound of formula (I) into a basic addition salt thereof.

3. A process for the preparation of a compound represented by the general formula (I) as defined in claim 1, or a basic addition salt thereof, which process comprises cyclodehydrating a compound of the general formula (XVIII)

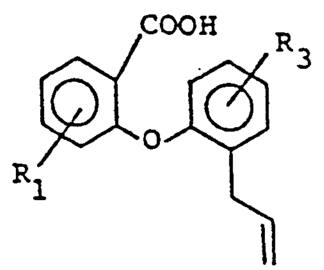

(XVIII)

where $R_1$ is hydrogen or is $R_1$ as defined in claim 1 and $R_3$ is as defined for $R_1$, not more than one of $R_1$ and $R_3$ being hydrogen; oxidising the obtained 4-allylxanthenone of the general formula (XIX)

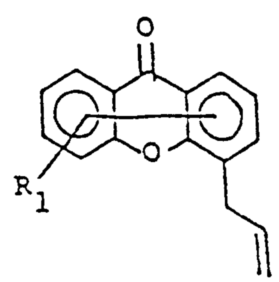

(XIX)

to the corresponding compound of formula (I) and, if desired, converting the compound of formula (I) into a basic addition salt thereof.

4. A process for the preparation of a compound of the general formula (I) as defined in claim 1, wherein $R_1$ is in the 5-position, or a basic addition salt thereof, which process comprises subjecting a compound of the general formula (XXII)

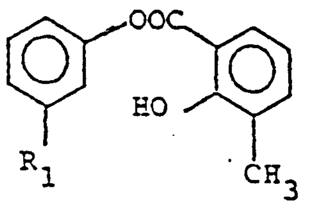

(XXII)

wherein $R_1$ is as defined in claim 1, to controlled pyrolysis, brominating the obtained 4-methylxanthenone of the general formula (XXIII)

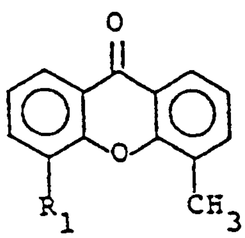

(XXIII)

reacting the obtained 4-bromomethylxanthenone with an inorganic cyanide; and hydrolysing the obtained xanthenone-4-acetonitrile to the corresponding compound of formula (I) and, if desired, converting the compound of formula (I) to a basic addition salt thereof.

**5.** A process according to any one of claims 1 to 4 where $R_1$ is 2-$CH_3$.

**6.** A process according to any one of claims 1 to 4 where $R_1$ is 2-$OCH_3$.

**7.** A process according to any one of claims 1 to 4 where $R_1$ is 5-$CH_3$.

**8.** A process according to any one of claims 1 to 4 where $R_1$ is 5-$OCH_3$

**9.** A process according to any one of claims 1 to 4 where $R_1$ is 5-Cl.

**10.** A process according to any one of claims 1 to 4 where $R_1$ is 6-$CH_3$.

**11.** A process according to any one of claims 1 to 4 where $R_1$ is 6-$OCH_3$.

**12.** A process according to any one of claims 1 to 4 where $R_1$ is 6-Cl.

**13.** A process according to any one of claims 1 to 4 where $R_1$ is 5,6-di$CH_3$.

**14.** A process according to any one of claims 1 to 4 where $R_1$ is the grouping -CH = CH-CH = CH- attached at the 5 and 6 positions to form an additional fused ring.

**15.** A process for the preparation of a pharmaceutical composition having antitumour activity which comprises bringing into admixture or conjunction at least one compound of the general formula (I) defined in claim 1, or a pharmaceutically-acceptable basic addition salt thereof, and one or more pharmaceutically acceptable carriers or diluents.

**16.** A process according to claim 15 wherein there is used a compound as defined in any one of claims 5 to 14, or a pharmaceutically acceptable basic addition salt thereof.

**17.** The use of xanthenone-4-acetic acid or of a compound of the general formula (I) defined in any one of claims 1 and 5 to 14, or a pharmaceutically acceptable basic addition salt thereof, for the manufacture of a medicament for use in the treatment of tumours.

**Patentansprüche**

**Patentansprüche für folgende Vertragsstaaten : AT, BE, CH, DE, FR, GB, IT, LI, LU, NL, SE**

1. Verbindung, wiedergegeben durch die allgemeine Formel (I)

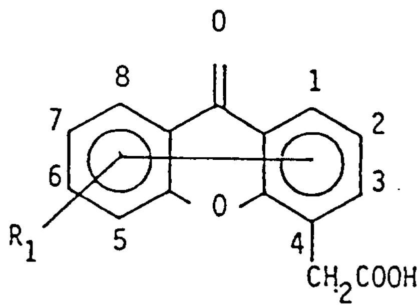

(I)

worin $R_1$ für bis zu zwei Gruppen der Gruppen ($C_1$- bis $C_5$-)Alkyl, Halogen, Phenyl, $CF_3$, CN, $NO_2$, $NH_2$, $CH_2COOH$, $OR_2$, OH, $NHCOR_2$, $NHSO_2R_2$, $SR_2$, $SO_2R_2$, $CH_2CONHR_2$ oder $NHR_2$, worin $R_2$ ein ($C_1$- bis $C_5$-) Alkylrest ist, der gegebenenfalls mit Hydroxy-, Amino- oder Methoxy-Funktionen substituiert ist, an einer beliebigen der Positionen 1 bis 8 steht, die verfügbar sind, wobei $R_1$ auch für die Substitution einer Aza-Gruppe (-N=-Gruppe) für eine oder zwei der Methin-Gruppen (-CH=-Gruppen) in den carbocyclischen Ringen stehen kann und zwei Gruppen $R_1$ an zwei verfügbaren benachbarten Positionen auch für die Gruppe -CH=CH-CH=CH- unter Bildung eines weiteren ankondensierten Benzolrings stehen können;
und die basischen Additionssalze davon.

2. Verbindung nach Anspruch 1 worin $R_1$ für 2-$CH_3$ steht.

3. Verbindung nach Anspruch 1 worin $R_1$ für 2-$OCH_3$ steht.

4. Verbindung nach Anspruch 1 worin $R_1$ für 5-$CH_3$ steht.

5. Verbindung nach Anspruch 1 worin $R_1$ für 5-$OCH_3$ steht.

6. Verbindung nach Anspruch 1 worin $R_1$ für 5-Cl steht.

7. Verbindung nach Anspruch 1 worin $R_1$ für 6-$CH_3$ steht.

8. Verbindung nach Anspruch 1 worin $R_1$ für 6-$OCH_3$ steht.

9. Verbindung nach Anspruch 1 worin $R_1$ für 6-Cl steht.

10. Verbindung nach Anspruch 1 worin $R_1$ für 5,6-di-$CH_3$ steht.

11. Verbindung nach Anspruch 1, worin $R_1$ für die Gruppe -CH=CH-CH=CH- steht, die an die Positionen 5 und 6 unter Bildung eines weiteren ankondensierten Rings gebunden ist.

12. Verfahren zur Herstellung einer durch die allgemeine Formel (I) nach Anspruch 1 wiedergegebenen Verbindung oder eines basischen Additionssalzes davon, wobei das Verfahren die folgenden Schritte umfaßt:

- Cyclodehydrieren einer substituierten Phenoxybenzoesäure der allgemeinen Formel (IV)

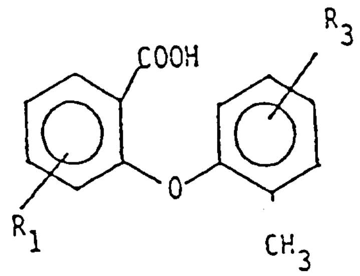

(IV)

worin $R_1$ Wasserstoff ist oder $R_1$ die in Patentanspruch 1 definierte Bedeutung aufweist und $R_3$ die für $R_1$ definierte Bedeutung aufweist und wobei nicht mehr als einer der Reste $R_1$ und $R_3$ Wasserstoff ist;

- Bromieren des erhaltenen 4-Methylxanthenons der allgemeinen Formel (VI)

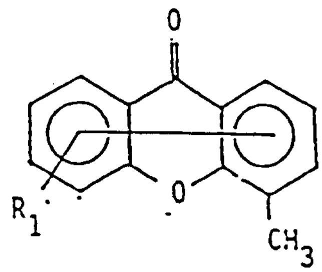

(VI)

- Umsetzen des erhaltenen 4-Brommethylxanthenons der allgemeinen Formel (VII)

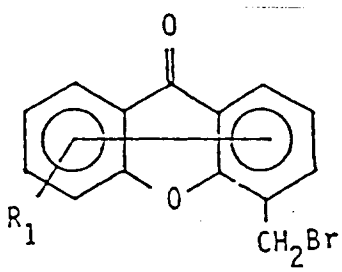

(VII)

mit einem anorganischen Cyanid; und

- Hydrolysieren des erhaltenen Xanthenon-4-acetonitrils der allgemeinen Formel (VIII)

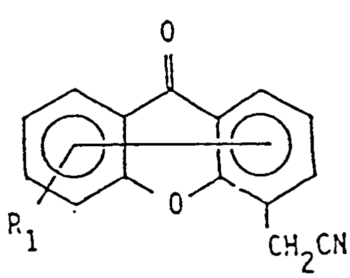

(VIII)

zu der entsprechenden Verbindung der Formel (I); und

- sofern erwünscht - Umwandeln der Verbindung der Formel (I) in eines ihrer basischen Additionssalze.

**13.** Verfahren zur Herstellung einer durch die allgemeine Formel (I) nach Anspruch 1 wiedergegebenen Verbindung oder eines basischen Additionssalzes davon, wobei das Verfahren die folgenden Schritte

umfaßt:

- Cyclodehydrieren einer substituierten Phenoxyphenylessigsäure der allgemeinen Formel (XI)

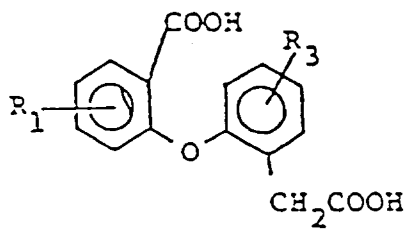

(XI)

worin $R_1$ Wasserstoff ist oder $R_1$ wie in Anspruch 1 definiert ist und $R_3$ die Bedeutung aufweist, wie sie für $R_1$ definiert ist, wobei nicht mehr als einer der Reste $R_1$ und $R_3$ Wasserstoff ist, zu der entsprechenden Verbindung der Formel (I); und

- sofern erwünscht - Umwandeln der Verbindung der Formel (I) in eines ihrer basischen Additionssalze.

**14.** Verfahren zur Herstellung einer durch die allgemeine Formel (I) nach Anspruch 1 wiedergegebenen Verbindung oder eines basischen Additionssalzes davon, wobei das Verfahren die folgenden Schritte umfaßt:

- Cyclodehydrieren einer Verbindung der allgemeinen Formel (XVIII)

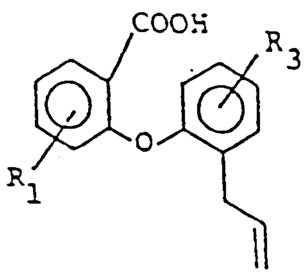

(XVIII)

worin $R_1$ Wasserstoff ist oder $R_1$ die in Anspruch 1 definierten Bedeutungen aufweist und $R_3$ eine Bedeutung aufweist, wie sie für $R_1$ definiert ist, wobei nicht mehr als einer der Reste $R_1$ und $R_3$ Wasserstoff ist;

- Oxydieren des erhaltenen 4-Allylxanthenons der allgemeinen Formel (XIX)

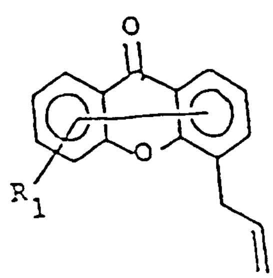

(XIX)

zu der entsprechenden Verbindung der Formel (I); und

- sofern erwünscht - Umwandeln der Verbindung der Formel (I) in eines ihrer basischen Additionssalze.

**15.** Verfahren zur Herstellung einer Verbindung der allgemeinen Formel (I) nach Anspruch 1, worin $R_1$ in der Position 5 steht, oder eines basischen Additionssalzes davon, wobei das Verfahren die folgenden Schritte umfaßt:

- Unterwerfen einer Verbindung der allgemeinen Formel (XXII)

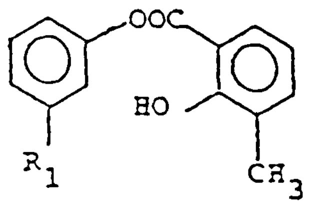

(XXII)

worin $R_1$ wie in Anspruch 1 definiert ist, einer gesteuerten Pyrolyse;
- Bromieren des erhaltenen 4-Methylxanthenons der allgemeinen Formel (XXIII)

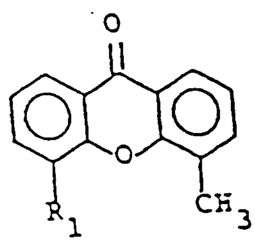

(XXIII)

- Umsetzen des erhaltenen 4-Brommethylxanthenons mit einem anorganischen Cyanid; und
- Hydrolysieren des erhaltenen Xanthenon-4-acetonitrils zu der entsprechenden Verbindung der Formel (I); und
- sofern erwünscht - Umwandeln der Verbindung der Formel (I) in eines ihrer basischen Additionssalze.

**16.** Pharmazeutische Zubereitung mit Antitumoraktivität, welche wenigstens eine Verbindung der allgemeinen Formel (I) nach Anspruch 1 oder ein pharmazeutisch annehmbares basisches Additionssalz davon und einen oder mehrere pharmazeutisch annehmbare Träger oder Verdünnungsmittel umfaßt.

**17.** Pharmazeutische Zubereitung mit Antitumoraktivität, welche eine Verbindung nach einem der Ansprüche 2 bis 11 oder ein pharmazeutisch annehmbares basisches Additionssalz davon oder einen oder mehrere pharmazeutisch annehmbare Träger oder Verdünnungsmittel umfaßt.

**18.** Verbindung der allgemeinen Formel (I) nach Anspruch 1 oder ein pharmazeutisch annehmbares basisches Additionssalz davon zur Verwendung bei der Behandlung von Tumoren.

**19.** Verbindung nach einem der Ansprüche 2 bis 11 oder ein pharmazeutisch annehmbares basisches Additionssalz davon zur Verwendung bei der Behandlung von Tumoren.

**20.** Verwendung der Xanthenon-4-essigsäure oder einer Verbindung der allgemeinen Formel (I) nach einem der Ansprüche 1 bis 11 oder eines pharmazeutisch annehmbaren basischen Additionssalzes davon zur Herstellung eines Medikamentes zur Behandlung von Tumoren.

**Patentansprüche für folgende Vertragsstaaten : ES, GR**

1. Verfahren zur Herstellung einer durch die allgemeine Formel (I) wiedergegebenen Verbindung

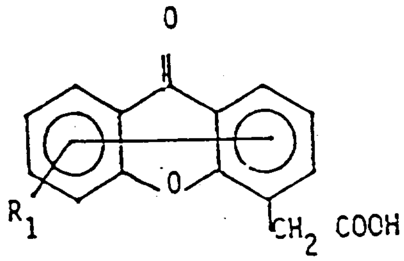

(I)

worin $R_1$ für bis zu zwei Gruppen der Gruppen ($C_1$- bis $C_5$-)Alkyl, Halogen, Phenyl, $CF_3$, CN, $NO_2$, $NH_2$, $CH_2COOH$, $OR_2$, OH, $NHCOR_2$, $NHSO_2R_2$, $SR_2$, $SO_2R_2$, $CH_2CONHR_2$ oder $NHR_2$, worin $R_2$ ein ($C_1$- bis $C_5$-) Alkylrest ist, der gegebenenfalls mit Hydroxy-, Amino- oder Methoxy-Funktionen substituiert ist, an einer beliebigen der Positionen 1 bis 8 steht, die verfügbar sind, wobei $R_1$ auch für die Substitution einer Aza-Gruppe` (-N=-Gruppe) für eine oder zwei der Methin-Gruppen (-CH=-Gruppen) in den carbocyclischen Ringen stehen kann und zwei Gruppen $R_1$ an zwei verfügbaren benachbarten Positionen auch für die Gruppe -CH=CH-CH=CH- unter Bildung eines weiteren ankondensierten Benzolrings stehen können,
oder eines basischen Additionssalzes davon, wobei das Verfahren die folgenden Schritte umfaßt:

- Cyclodehydrieren einer substituierten Phenoxybenzoesäure der allgemeinen Formel (IV)

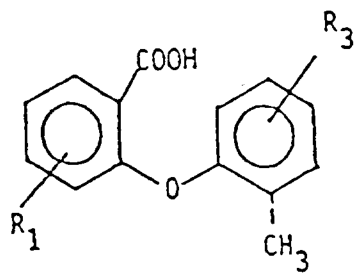

(IV)

worin $R_1$ Wasserstoff ist oder $R_1$ die oben definierte Bedeutung aufweist und $R_3$ die für $R_1$ definierte Bedeutung aufweist und wobei nicht mehr als einer der Reste $R_1$ und $R_3$ Wasserstoff ist;

- Bromieren des erhaltenen 4-Methylxanthenons der allgemeinen Formel (VI)

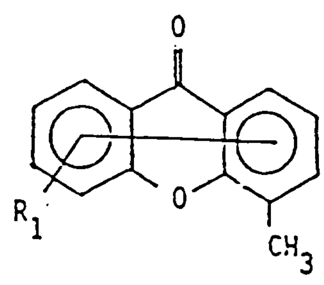

(VI)

- Umsetzen des erhaltenen 4-Brommethylxanthenons der allgemeinen Formel (VII)

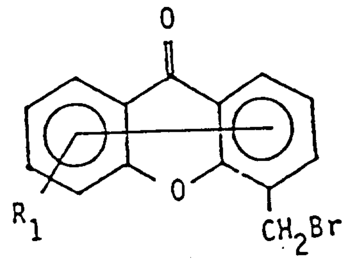

(VII)

mit einem anorganischen Cyanid; und
- Hydrolysieren des erhaltenen Xanthenon-4-acetonitrils der allgemeinen Formel (VIII)

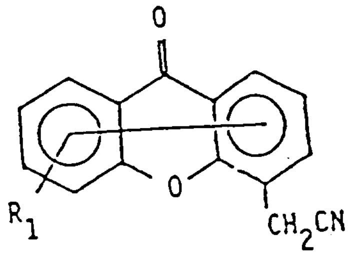

(VIII)

zu der entsprechenden Verbindung der Formel (I); und
- sofern erwünscht - Umwandeln der Verbindung der Formel (I) zu einem ihrer basischen Additionssalze.

**2.** Verfahren zur Herstellung einer durch die allgemeine Formel (I) nach Anspruch 1 wiedergegebenen Verbindung oder eines basischen Additionssalzes davon, wobei das Verfahren die folgenden Schritte umfaßt:
- Cyclodehydrieren einer substituierten Phenoxyphenylessigsäure der allgemeinen Formel (XI)

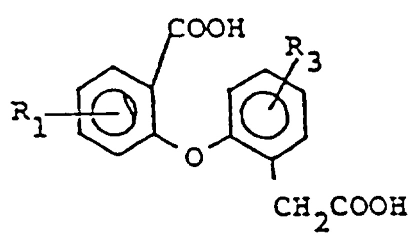

(XI)

worin $R_1$ Wasserstoff ist oder $R_1$ wie in Anspruch 1 definiert ist und $R_3$ die Bedeutung aufweist, wie sie für $R_1$ definiert ist, wobei nicht mehr als einer der Reste $R_1$ und $R_3$ Wasserstoff ist, zu der entsprechenden Verbindung der Formel (I); und
- sofern erwünscht - Umwandeln der Verbindung der Formel (I) in eines ihrer basischen Additionssalze.

**3.** Verfahren zur Herstellung einer durch die allgemeine Formel (I) nach Anspruch 1 wiedergegebenen Verbindung oder eines basischen Additionssalzes davon, wobei das Verfahren die folgenden Schritte umfaßt:

- Cyclodehydrieren einer Verbindung der allgemeinen Formel (XVIII)

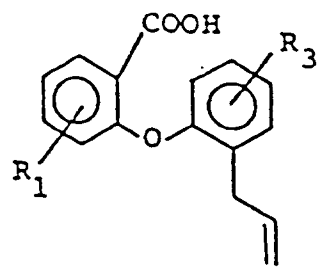

(XVIII)

worin $R_1$ Wasserstoff ist oder $R_1$ die in Anspruch 1 definierten Bedeutungen aufweist und $R_3$ eine Bedeutung aufweist, wie sie für $R_1$ definiert ist, wobei nicht mehr als einer der Reste $R_1$ und $R_3$ Wasserstoff ist;

- Oxydieren des erhaltenen 4-Allylxanthenons der allgemeinen Formel (XIX)

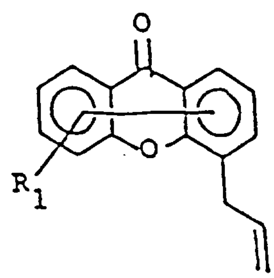

(XIX)

zu der entsprechenden Verbindung der Formel (I); und

- sofern erwünscht - Umwandeln der Verbindung der Formel (I) in eines ihrer basischen Additionssalze.

**4.** Verfahren zur Herstellung einer Verbindung der allgemeinen Formel (I) nach Anspruch 1, worin $R_1$ in der Position 5 steht, oder eines basischen Additionssalzes davon, wobei das Verfahren die folgenden Schritte umfaßt:

- Unterwerfen einer Verbindung der allgemeinen Formel (XXII)

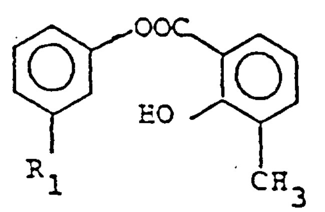

(XXII)

worin $R_1$ wie in Anspruch 1 definiert ist, einer gesteuerten Pyrolyse;

- Bromieren des erhaltenen 4-Methylxanthenons der allgemeinen Formel (XXIII)

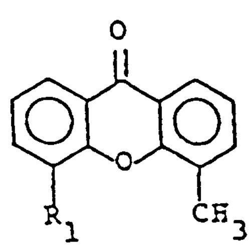

(XXIII)

- Umsetzen des erhaltenen 4-Brommethylxanthenons mit einem anorganischen Cyanid; und
- Hydrolysieren des erhaltenen Xanthenon-4-acetonitrils zu der entsprechenden Verbindung der Formel (I); und
- sofern erwünscht - Umwandeln der Verbindung der Formel (I) in eines ihrer basischen Additionssalze.

**5.** Verfahren nach einem der Ansprüche 1 bis 4, worin $R_1$ für 2-$CH_3$ steht.

**6.** Verfahren nach einem der Ansprüche 1 bis 4, worin $R_1$ für 2-$OCH_3$ steht.

**7.** Verfahren nach einem der Ansprüche 1 bis 4, worin $R_1$ für 5-$CH_3$ steht.

**8.** Verfahren nach einem der Ansprüche 1 bis 4, worin $R_1$ für 5-$OCH_3$ steht.

**9.** Verfahren nach einem der Ansprüche 1 bis 4, worin $R_1$ für 5-Cl steht.

**10.** Verfahren nach einem der Ansprüche 1 bis 4, worin $R_1$ für 6-$CH_3$ steht.

**11.** Verfahren nach einem der Ansprüche 1 bis 4, worin $R_1$ für 6-$OCH_3$ steht.

**12.** Verfahren nach einem der Ansprüche 1 bis 4, worin $R_1$ für 6-Cl steht.

**13.** Verfahren nach einem der Ansprüche 1 bis 4, worin $R_1$ für 5,6-di-$CH_3$ steht.

**14.** Verfahren nach einem der Ansprüche 1 bis 4, worin $R_1$ für die Gruppe -CH = CH-CH = CH- steht, die an die Positionen 5 und 6 unter Bildung eines weiteren ankondensierten Rings gebunden ist.

**15.** Verfahren zur Herstellung einer pharmazeutischen Zubereitung mit Antitumoractivität, welches den Schritt umfaßt, daß man wenigstens eine Verbindung der allgemeinen Formel (I) nach Anspruch 1 oder ein pharmazeutisch annehmbares basisches Additionssalz davon und einen oder mehrere pharmazeutisch annehmbare Träger oder Verdünnungsmittel in Mischung oder Vereinigung miteinander bringt.

**16.** Verfahren nach Anspruch 15, wobei man eine Verbindung, wie sie in einem der Ansprüche 5 bis 14 definiert ist oder ein pharmazeutisch annehmbares basisches Additionssalz davon verwendet.

**17.** Verwendung der Xanthenon-4-essigsäure oder einer Verbindung der allgemeinen Formel (I), wie sie in einem der Ansprüche 1 und 5 bis 14 definiert ist, oder eines pharmazeutisch annehmbaren basischen Additionssalzes davon, zur Herstellung eines Arzneimittels zur Verwendung bei der Behandlung von Tumoren.

**Revendications**
**Revendications pour les Etats contractants suivants : AT, BE, CH, DE, FR, GB, IT, LI, LU, NL, SE**

**1.** Composé représenté par la formule générale (I)

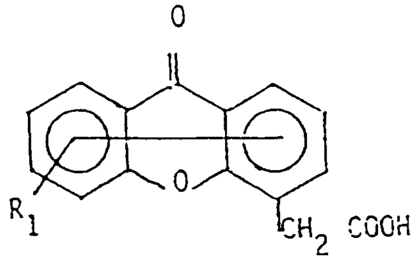

(I)

dans laquelle $R_1$ représente jusqu'à deux des groupes alkyle en $C_1$-$C_5$, halogéno, phényle, $CF_3$, CN, $NO_2$, $NH_2$, $CH_2COOH$, $OR_2$, OH, $NHCOR_2$, $NHSO_2R_2$, $SR_2$, $SO_2R_2$, $CH_2CONHR_2$ ou $NHR_2$ (où $R_2$ est un groupe alkyle en $C_1$-$C_5$ facultativement substitué par des fonctions hydroxyle, amino ou méthoxy)

fixé(s) à une ou deux quelconques des positions 1 à 8 qui sont libres, $R_1$ peut également représenter la substitution d'un groupe aza (-N=) à un ou deux des groupes méthyne (-CH=) dans les carbocycles, et deux $R_1$ fixés à deux quelconques positions adjacentes libres peuvent également représenter le groupement -CH=CH-CH=CH- formant un noyau benzénique accolé supplémentaire ; et ses sels d'addition de base.

**2.** Composé selon la revendication 1, où $R_1$ est 2-$CH_3$.

**3.** Composé selon la revendication 1, où $R_1$ est 2-$OCH_3$.

**4.** Composé selon la revendication 1, où $R_1$ est 5-$CH_3$.

**5.** Composé selon la revendication 1, où $R_1$ est 5-$OCH_3$.

**6.** Composé selon la revendication 1, où $R_1$ est 5-Cl.

**7.** Composé selon la revendication 1, où $R_1$ est 6-$CH_3$.

**8.** Composé selon la revendication 1, où $R_1$ est 6-$OCH_3$.

**9.** Composé selon la revendication 1, où $R_1$ est 6-Cl.

**10.** Composé selon la revendication 1, où $R_1$ est 5,6-di$CH_3$.

**11.** Composé selon la revendication 1, ou $R_1$ est le groupement -CH=CH-CH=CH- fixé aux positions 5 et 6 en formant un noyau accolé supplémentaire.

**12.** Procédé pour la préparation d un composé représenté par la formule générale (I) tel que défini dans la revendication 1, ou d'un sel d'addition de base de ce composé, lequel procédé consiste à déshydrocycliser un acide phénoxybenzoïque substitué de formule générale (IV)

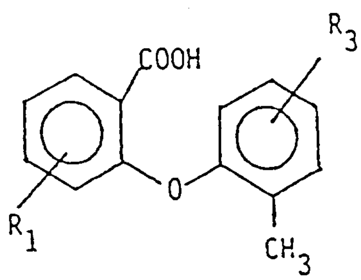

(IV)

où $R_1$ est l'hydrogène ou est $R_1$ tel que défini dans la revendication 1 et $R_3$ est tel que défini pour $R_1$, l'un au plus de $R_1$ et $R_3$ étant l'hydrogène, bromer la 4-méthyl-xanthénone obtenue de formule générale (VI)

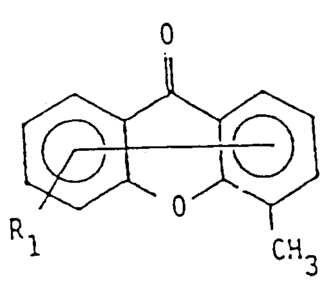

(VI)

faire réagir la 4-bromométhylxanthénone obtenue de formule générale (VII)

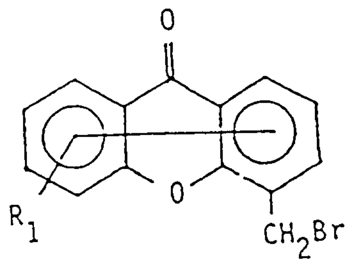

(VII)

avec un cyanure minéral, et hydrolyser le xanthénone-4-acétonitrile obtenu de formule générale (VIII)

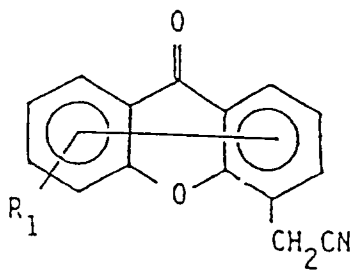

(VIII)

en le composé correspondant de formule (I) et, facultativement, convertir le composé de formule (I) en un de ses sels d'addition de base.

**13.** Procédé pour la préparation d'un composé représenté par la formule générale (I) tel que défini dans la revendication 1, ou d'un sel d'addition de base de ce composé, lequel procédé consiste à déshydrocycliser un acide phénoxyphénylacétique substitué de formule générale (XI)

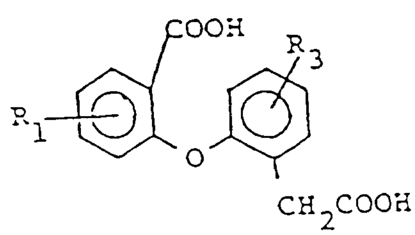

(XI)

où $R_1$ est l'hydrogène ou est $R_1$ tel que défini dans la revendication 1 et $R_3$ est tel que défini pour $R_1$, l'un au plus de $R_1$ et $R_3$ étant l'hydrogène, en le composé correspondant de formule (I) et, facultativement, convertir le composé de formule (I) en un de ses sels d'addition de base.

**14.** Procédé pour la préparation d'un composé représenté par la formule générale (I) tel que défini dans la revendication 1, ou d'un sel d'addition de base de ce composé, lequel procédé consiste à déshydrocycliser un composé de formule générale (XVIII)

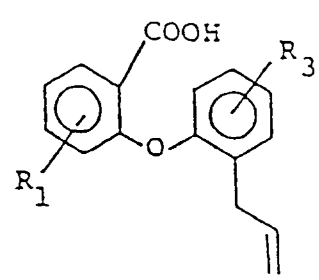

(XVIII)

où $R_1$ est l'hydrogène ou est $R_1$ tel que défini dans la revendication 1 et $R_3$ est tel que défini pour $R_1$, l'un au plus de $R_1$ et $R_3$ étant l'hydrogène, oxyder la 4-allyl-xanthénone obtenue de formule générale

(XIX)

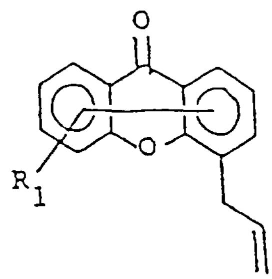

(XIX)

en le composé correspondant de formule (I) et, facultativement, convertir le composé de formule (I) en un de ses sels d'addition de base.

**15.** Procédé pour la préparation d un composé représenté par la formule générale (I) tel que défini dans la revendication 1, où $R_1$ est à la position 5, ou d'un sel d'addition de base de ce composé, lequel procédé consiste à soumettre à une pyrolyse ménagée un composé de formule générale (XXII)

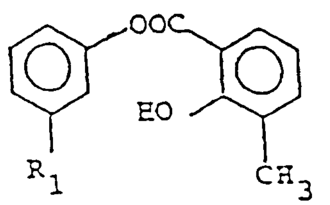

(XXII)

où $R_1$ est tel que défini dans la revendication 1, bromer la 4-méthylxanthénone obtenue de formule générale (XXIII)

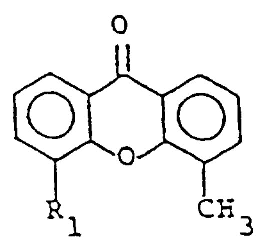

(XXIII)

faire réagir la 4-bromométhylxanthénone obtenue avec un cyanure minéral, et hydrolyser le xanthénone-4-acétonitrile obtenu en le composé correspondant de formule (I) et, facultativement, convertir le composé de formule (I) en un de ses sels d'addition de base.

**16.** Composition pharmaceutique douée d'activité antitumorale, qui comprend au moins un composé de formule générale (I) défini dans la revendication 1, ou un sel d'addition de base pharmaceutiquement acceptable de ce composé, et un ou plusieurs supports ou diluants pharmaceutiquement acceptables.

**17.** Composition pharmaceutique douée d'activité antitumorale, qui comprend un composé selon l'une quelconque des revendications 2 à 11, ou un sel d'addition de base pharmaceutiquement acceptable de ce composé, et un ou plusieurs supports ou diluants pharmaceutiquement acceptables.

**18.** Composé de formule générale (I) défini dans la revendication 1, ou sel d'addition de base pharmaceutiquement acceptable de ce composé, pour son utilisation dans le traitement de tumeurs.

**19.** Composé selon l'une quelconque des revendications 2 à 11, ou sel d'addition de base pharmaceutiquement acceptable de ce composé, pour son utilisation dans le traitement de tumeurs.

**20.** Utilisation de l'acide xanthénone-4-acétique ou d'un composé de formule générale (I) tel que revendiqué dans l'une quelconque des revendications 1 à 11, ou d'un de leurs sels d'addition de base pharmaceutiquement acceptables, pour la fabrication d'un médicament destiné à être utilisé dans le traitement de tumeurs.

**Revendications pour les Etats contractants suivants : ES, GR**

**1.** Procédé pour la préparation d'un composé représenté par la formule générale (I)

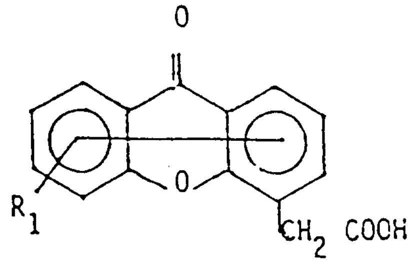

(I)

dans laquelle $R_1$ représente jusqu'à deux des groupes alkyle en $C_1$-$C_5$, halogéno, phényle, $CF_3$, CN, $NO_2$, $NH_2$, $CH_2COOH$, $OR_2$, OH, $NHCOR_2$, $NHSO_2R_2$, $SR_2$, $SO_2R_2$, $CH_2CONHR_2$ ou $NHR_2$ (où $R_2$ est un groupe alkyle en $C_1$-$C_5$ facultativement substitué par des fonctions hydroxyle, amino ou méthoxy) fixé(s) à une ou deux quelconques des positions 1 à 8 qui sont libres, $R_1$ peut également représenter la substitution d'un groupe aza (-N=) à un ou deux des groupes méthyne (-CH=) dans les carbocycles, et deux $R_1$ fixés à deux quelconques positions adjacentes libres peuvent également représenter le groupement -CH=CH-CH=CH- formant un noyau benzénique accolé supplémentaire ;
ou d'un sel d'addition de base de ce composé, lequel procédé consiste à déshydrocycliser un acide phénoxybenzoïque substitué de formule générale (IV)

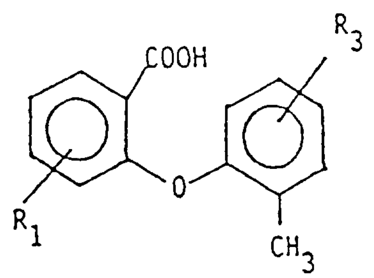

(IV)

où $R_1$ est l'hydrogène ou est $R_1$ tel que défini ci-dessus et $R_3$ est tel que défini pour $R_1$, l'un au plus de $R_1$ et $R_3$ étant l'hydrogène, bromer la 4-méthylxanthénone obtenue de formule générale (VI)

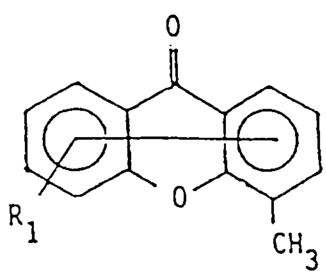

(VI)

faire réagir la 4-bromométhylxanthénone obtenue de formule générale (VII)

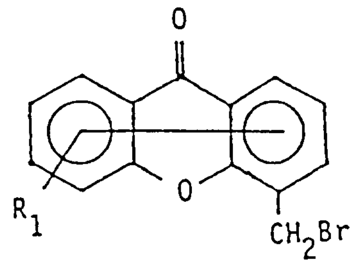

(VII)

avec un cyanure minéral, et hydrolyser le xanthénone-4-acétonitrile obtenu de formule générale (VIII)

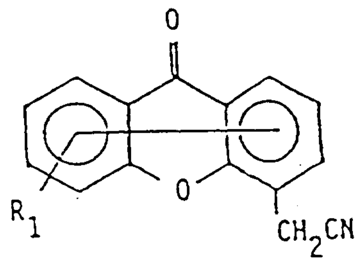

(VIII)

en le composé correspondant de formule (I) et, facultativement, convertir le composé de formule (I) en un de ses sels d'addition de base.

**2.** Procédé pour la préparation d'un composé représenté par la formule générale (I) tel que défini dans la revendication 1, ou d'un sel d'addition de base de ce composé, lequel procédé consiste à déshydrocycliser un acide phénoxyphénylacétique substitué de formule générale (XI)

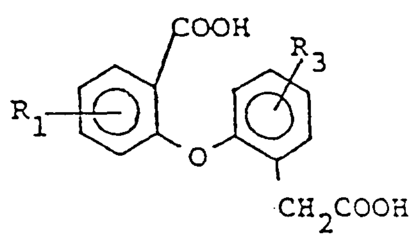

(XI)

où $R_1$ est l'hydrogène ou est $R_1$ tel que défini dans la revendication 1 et $R_3$ est tel que défini pour $R_1$, l'un au plus de $R_1$ et $R_3$ étant l'hydrogène, en le composé correspondant de formule (I) et, facultativement, convertir le composé de formule (I) en un de ses sels d'addition de base.

**3.** Procédé pour la préparation d un composé représenté par la formule générale (I) tel que défini dans la revendication 1, ou d'un sel d'addition de base de ce composé, lequel procédé consiste à déshydrocycliser un composé de formule générale (XVIII)

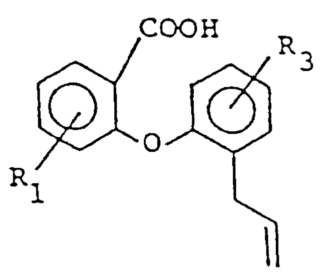

(XVIII)

où $R_1$ est l'hydrogène ou est $R_1$ tel que défini dans la revendication 1 et $R_3$ est tel que défini pour $R_1$, l'un au plus de $R_1$ et $R_3$ étant l'hydrogène, oxyder la 4-allyl-xanthénone obtenue de formule générale

(XIX)

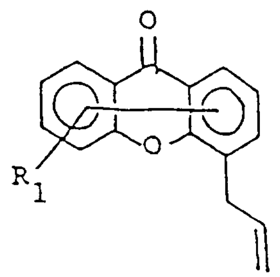

(XIX)

en le composé correspondant de formule (I) et, facultativement, convertir le composé de formule (I) en un de ses sels d'addition de base.

**4.** Procédé pour la préparation d'un composé représenté par la formule générale (I) tel que défini dans la revendication 1, où $R_1$ est à la position 5, ou d'un sel d'addition de base de ce composé, lequel procédé consiste à soumettre à une pyrolyse ménagée un composé de formule générale (XXII)

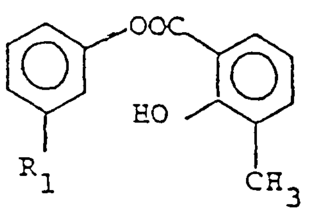

(XXII)

où $R_1$ est tel que défini dans la revendication 1, bromer la 4-méthylxanthénone obtenue de formule générale (XXIII)

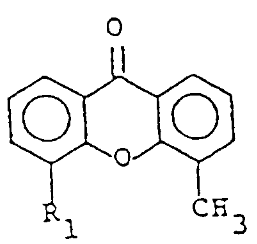

(XXIII)

faire réagir la 4-bromométhylxanthénone obtenue avec un cyanure minéral, et hydrolyser le xanthenone-4-acétonitrile obtenu en le composé correspondant de formule (I) et, facultativement, convertir le composé de formule (I) en un de ses sels d'addition de base.

**5.** Procédé selon l'une quelconque des revendications 1 à 4, où $R_1$ est 2-$CH_3$.

**6.** Procédé selon l'une quelconque des revendications 1 à 4, où $R_1$ est 2-$OCH_3$.

**7.** Procédé selon l une quelconque des revendications 1 à 4, où $R_1$ est 5-$CH_3$.

**8.** Procédé selon l une quelconque des revendications 1 à 4, où $R_1$ est 5-$OCH_3$.

**9.** Procédé selon l'une quelconque des revendications 1 à 4, où $R_1$ est 5-Cl.

**10.** Procédé selon l'une quelconque des revendications 1 à 4, où $R_1$ est 6-$CH_3$.

**11.** Procédé selon l'une quelconque des revendications 1 à 4, où $R_1$ est 6-$OCH_3$.

**12.** Procédé selon l'une quelconque des revendications 1 à 4, où $R_1$ est 6-Cl.

**13.** Procédé selon l'une quelconque des revendications 1 à 4, où $R_1$ est 5,6-di$CH_3$.

**14.** Procédé selon l'une quelconque des revendications 1 à 4, où $R_1$ est le groupement -CH = CH-CH = CH- fixé aux positions 5 et 6 en formant un noyau accolé supplémentaire.

**15.** Procédé pour la préparation d'une composition pharmaceutique douée d'activité antitumorale, qui consiste mettre sous forme de mélange ou d'association au moins un composé de formule générale (I) défini dans la revendication 1, ou un sel d'addition de base pharmaceutiquement acceptable de ce composé, et un ou plusieurs supports ou diluants pharmaceutiquement acceptables.

**16.** Procédé selon la revendication 15, dans lequel est utilisé un composé tel que défini dans l'une quelconque des revendications 5 à 14, ou un sel d'addition de base pharmaceutiquement acceptable de ce composé.

**17.** Utilisation de l'acide xanthénone-4-acétique ou d'un composé de formule générale (I) défini dans l'une quelconque des revendications 1 et 5 à 14, ou d'un de leurs sels d'addition d'acide pharmaceutiquement acceptables, pour la fabrication d'un médicament destiné à être utilisé dans le traitement de tumeurs.